# EUROPEAN PATENT SPECIFICATION

(11) **EP 4 456 787 B1**
(45) Date of publication and mention of the grant of the patent: **15.07.2026**
(21) Application number: 23735196.0
(22) Date of filing: 02.01.2023
(51) Int. Cl.: A61N 1/378, A61N 1/05, A61N 1/372, A61N 1/08

(54) **DEVICE AND METHOD FOR EVALUATING COUPLING WITH AN IMPLANTABLE DEVICE**
VORRICHTUNG UND VERFAHREN ZUR BEURTEILUNG DER KOPPLUNG MIT EINER IMPLANTIERBAREN VORRICHTUNG
DISPOSITIF ET PROCÉDÉ D'ÉVALUATION DE COUPLAGE AVEC UN DISPOSITIF IMPLANTABLE

(30) Priority: 02.01.2022 US 202263295907 P
(43) Date of publication of application: 06.11.2024
(73) Proprietor: Exact Neuro LLC, Napa / California / 94558 (US)
(72) Inventor: Glukhovsky, Arkady, Nesher (IL)
(74) Representative: Murgitroyd & Company
(86) International application number: PCT/US2023/060007
(87) International publication number: WO 2023/130132

(56) References cited:
- WO-A2-2008/012523
- US-A1- 2012 041 279
- US-A1- 2014 172 053
- US-A1- 2014 214 125
- US-A1- 2017 021 172
- US-A1- 2021 346 704

## Description

### BACKGROUND

### Technical Field

The present disclosure relates generally to medical devices and treatments, and more particularly, to a partially implantable system for delivery of transcutaneous current.

### Introduction

Implantable devices can be implanted into a tissue of a subject, such as a human patient, to apply electrical current to nerves or tissue (e.g., to stimulate nerves or tissue) and to record or sense neural signals emanating from the central and peripheral nervous system. Implantable devices can be used for a variety of clinical indications, including delivering electrical stimulation and/or sensing to treat pain, incontinence, motor response, headache and migraine, and other indications.

Some systems may use an external device, such as an external generator, to apply transcutaneous current to the skin of a subject and use an implantable device beneath the skin of the subject to provide a path for the applied transcutaneous current. Once the implantable device is implanted beneath the skin of the subject, various occurrences may impact the coupling between the implantable device and the external generator. For example, one or more parts of the implantable device may stop operating correctly, or the tissue at the implantation site may scar or otherwise change in a manner that impacts the coupling. Furthermore, if the external device is not positioned properly on the skin of the subject with respect to the implantable device, the current applied by the external device may not pass through the implantable device. The implantable device may not be able to communicate whether the coupling with the external device has been impacted or when the external device is properly aligned with the implantable device, as it may be desired to minimize the size of the implantable device, so it may not be desirable to include components and/or utilize power for communicating information back to the external generator.

WO2008/012523 discloses a method and a device for evaluating coupling of an implantable device with an external generator.

### SUMMARY

The invention is defined by the independent claims. The following presents a simplified summary of one or more aspects in order to provide a basic understanding of such aspects. This summary is not an extensive overview of all contemplated aspects, and is intended to neither identify key or critical elements of all aspects nor delineate the scope of any or all aspects. Its sole purpose is to present some concepts of one or more aspects in a simplified form as a prelude to the more detailed description that is presented later.

**In** an aspect of the disclosure, a method of evaluating coupling between an implantable device and an external generator is provided, as defined in appended claim 1. The method may be performed by an external generator. The methodincludes applying a first transcutaneous current to the skin of the subject between the first skin electrode and the second skin electrode utilizing a first electrode surface area; determining a first impedance value based on the first current; applying a second transcutaneous current to the skin of the subject between the first skin electrode and the second skin electrode utilizing a second electrode surface area; determining a second impedance value based on the second current; applying a third transcutaneous current to the skin of the subject between the first skin electrode and the second skin electrode utilizing a third electrode surface area, the third transcutaneous current being different than the first transcutaneous current; determining a third impedance value based on the third current; and determining an implant current or a pick up ratio of the implantable device based on the first impedance value, the second impedance value, and the third impedance value.

**In** some aspects, the first transcutaneous current is applied in a first direction, the second transcutaneous current is applied in the first direction, and the third transcutaneous current is applied in a second direction different than the first duration.

**In** some aspects, the implantable device comprises a rectifier, the first transcutaneous current does not cause the rectifier to conduct based on the first direction, the second transcutaneous current does not cause the rectifier to conduct based on the first direction, and the third transcutaneous current does cause the rectifier to conduct based on the second direction.

**In** some aspects, the rectifier is a diode.

In some aspects, the first transcutaneous current and the second transcutaneous current have a first amplitude, and the third transcutaneous current has a second amplitude, the second amplitude being different than the first amplitude.

In some aspects, the implantable device comprises a rectifier, wherein the first transcutaneous current and the second transcutaneous current do not cause the rectifier to conduct based on the first amplitude, and wherein the third transcutaneous current does cause the rectifier to conduct based on the second amplitude.

In some aspects, the rectifier is a diode.

In some aspects, the first transcutaneous current is applied for a first duration, the second transcutaneous current is applied for the first duration, and the third transcutaneous current is applied for a second duration, the second duration being different than the first duration.

In some aspects, the first transcutaneous current, the second transcutaneous current, and the third transcutaneous current are alternating currents.

In some aspects, the implantable device comprises a capacitor that may be charged by current passing through the implantable device, applying the first transcutaneous current over the first duration causes the capacitor to have a first effective impedance, applying the second transcutaneous current over the first duration causes the capacitor to have a second effective impedance, applying the third transcutaneous current over the second duration causes the capacitor to have a third effective impedance, and the third effective impedance is lower than the first effective impedance and the second effective impedance.

In some aspects, determining the implant current or the pick up ratio of the implantable device comprises determining a first electrode-skin impedance value corresponding to the first electrode surface area, a second electrode-skin impedance value corresponding to the second electrode surface area, a tissue path impedance value, and an implant path impedance value.

In some aspects, determining the first electrode-skin impedance value or the second electrode-skin impedance value comprises determining a ratio between the first electrode surface area and the second electrode surface area based on a capacitive component of the skin of the subject.

In some aspects, determining the implant current or the pick up ratio comprises determining a first electrode-skin impedance value corresponding to the first electrode surface area, a second electrode-skin impedance value corresponding to the second electrode surface area, and a tissue path impedance value based on the first impedance value and the second impedance value, and determining an implant path impedance value based on the tissue path impedance value, the first electrode-skin impedance value, the second electrode-skin impedance value, and the third impedance value.

In some aspects, the method may include outputting an implant current indication based on the implant current or the pick up ratio.

In another aspect of the disclosure, an external generator is provided, as defined in appended claim 9. The external generator includes a current generator; a first skin electrode coupled to the current generator; a second skin electrode coupled to the current generator; and at least one processor coupled to the current generator, wherein: the current generator is configured to apply a first transcutaneous current to a skin of a subject between the first skin electrode and the second skin electrode utilizing a first electrode surface area, the processor is configured to determine a first impedance value based on the first current, the current generator is configured to apply a second transcutaneous current to the skin of the subject between the first skin electrode and the second skin electrode utilizing a second electrode surface area, the processor is configured to determine a second impedance value based on the second current, the current generator is configured to apply a third transcutaneous current to the skin of the subject between the first skin electrode and the second skin electrode utilizing a third electrode surface area, the third transcutaneous current being different than the first transcutaneous current, the processor is configured to determine a third impedance value based on the third current, and the processor is configured to determine an implant current or a pick up ratio of an implantable device implanted beneath the skin of the subject based on the first impedance value, the second impedance value, and the third impedance value.

In some aspects, the current generator is configured to apply the first transcutaneous current in a first direction, apply the second transcutaneous current in the first direction, and apply the third transcutaneous current in a second direction different than the first duration.

In some aspects, the implantable device comprises a rectifier, the first transcutaneous current does not cause the rectifier to conduct based on the first direction, the second transcutaneous current does not cause the rectifier to conduct based on the first direction, and the third transcutaneous current does cause the rectifier to conduct based on the second direction.

In some aspects, the rectifier is a diode.

In some aspects, the first transcutaneous current and the second transcutaneous current have a first amplitude, and the third transcutaneous current has a second amplitude, the second amplitude being different than the first amplitude.

In some aspects, the implantable device comprises a rectifier, wherein the first transcutaneous current and the second transcutaneous current do not cause the rectifier to conduct based on the first amplitude, and wherein the third transcutaneous current does cause the rectifier to conduct based on the second amplitude.

In some aspects, the rectifier is a diode.

In some aspects, the current generator is configured to apply the first transcutaneous current for a first duration, apply the second transcutaneous current for the first duration, and apply the third transcutaneous current for a second duration, the second duration being different than the first duration.

In some aspects, the first transcutaneous current, the second transcutaneous current, and the third transcutaneous current are alternating currents.

In some aspects, the implantable device comprises a capacitor that may be charged by current passing through the implantable device, applying the first transcutaneous current over the first duration causes the capacitor to have a first effective impedance, applying the second transcutaneous current over the first duration causes the capacitor to have a second effective impedance, applying the third transcutaneous current over the second duration causes the capacitor to have a third effective impedance, and the third effective impedance is lower than the first effective impedance and the second effective impedance.

In some aspects, determining the implant current or the pick up ratio of the implantable device comprises determining a first electrode-skin impedance value corresponding to the first electrode surface area, a second electrode-skin impedance value corresponding to the second electrode surface area, a tissue path impedance value, and an implant path impedance value.

In some aspects, determining the first electrode-skin impedance value or the second electrode-skin impedance value comprises determining a ratio between the first electrode surface area and the second electrode surface area based on a capacitive component of the skin of the subject.

In some aspects, determining the implant current or the pick up ratio comprises determining a first electrode-skin impedance value corresponding to the first electrode surface area, a second electrode-skin impedance value corresponding to the second electrode surface area, and a tissue path impedance value based on the first impedance value and the second impedance value, and determining an implant path impedance value based on the tissue path impedance value, the first electrode-skin impedance value, the second electrode-skin impedance value, and the third impedance value.

In some aspects, the processor is configured to output an implant current indication.

To the accomplishment of the foregoing and related ends, the one or more aspects comprise the features hereinafter fully described and particularly pointed out in the claims. The following description and the annexed drawings set forth in detail certain illustrative features of the one or more aspects. These features are indicative, however, of but a few of the various ways in which the principles of various aspects may be employed, and this description is intended to include all such aspects and their equivalents.

### BRIEF DESCRIPTION OF THE DRAWINGS

FIG. 1 is a diagram illustrating a system for applying transcutaneous current.
FIG. 2 is a diagram illustrating an embodiment of a rectification circuit for the implantable device described with respect to FIG. 1.
FIG. 3A is a diagram illustrating an electrical model representing a system for applying transcutaneous current in-vivo.
FIG. 3B is a diagram illustrating an electrical model representing the system for applying transcutaneous current while the implanted component with different forward and backward impedances is conducting.
FIG. 3C is a diagram illustrating an electrical model representing the system for applying transcutaneous current while the implanted component with different forward and backward impedances is not conducting.
FIG. 4 is a flowchart illustrating a method of detecting functionality in an implantable device.
FIG. 5 is a diagram illustrating a multi-channel system for applying transcutaneous current.
FIG. 6 is a circuit diagram of an implantable device for applying multi-channel stimulation.
FIG. 7A is a diagram illustrating an electrical model representing a multi-channel system for applying transcutaneous current in-vivo
FIG. 7B is a diagram illustrating an electrical model representing the system for applying transcutaneous current to the implantable device while the first component of the first channel is conducting and the second component of the second channel is not conducting.
FIG. 7C is a diagram illustrating an electrical model representing the system for applying transcutaneous current to the implantable device while the second component of the second channel is conducting and the first component of the first channel is not conducting.
FIG. 8 is a flowchart illustrating a method of detecting functionality in a multi-channel implantable device.
FIG. 9 is a flowchart illustrating a method of determining functionality in a multi-channel implantable device.
FIG. 10 is a block diagram illustrating an external generator.
FIG. 11 is a block diagram illustrating an external generator and an implant monitor.
FIG. 12 is a diagram of an external generator capable of applying current using different electrode surface areas.
FIG. 13 is a diagram of an external generator utilizing a frequency selected segmented electrode.
FIG. 14 is a flowchart illustrating a method of determining the current passing through an implantable device.
FIG. 15 is a flowchart illustrating a method of determining the current passing through an implantable device.
FIG. 16 is a flowchart illustrating a method of determining the current passing through an implantable device.
FIG. 17 is a block diagram illustrating an external generator.
FIG. 18 is a block diagram illustrating an external generator and an electrode adapter.

### DETAILED DESCRIPTION

The detailed description set forth below in connection with the appended drawings is intended as a description of various configurations and is not intended to represent the only configurations in which the concepts described herein may be practiced. The detailed description includes specific details for the purpose of providing a thorough understanding of various concepts. However, it will be apparent to those skilled in the art that these concepts may be practiced without these specific details. In some instances, well known structures and components may be shown in block diagram form in order to avoid obscuring such concepts.

Several aspects of medical devices will now be presented with reference to various apparatus and methods.

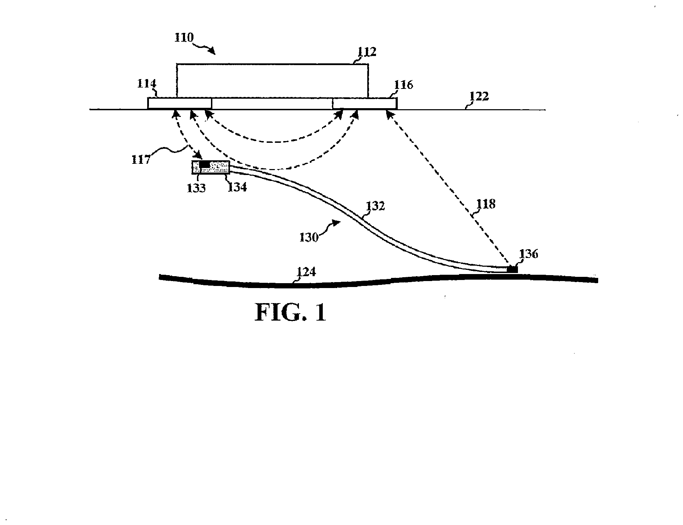

FIG. 1 is a diagram illustrating a system for applying transcutaneous current. The system includes an external generator 110 and an implantable device 130. The external generator 110 includes a device housing 112, a source electrode 114, and a return electrode 116. source electrode The source electrode 114 and the return electrode 116 may be similar or identical electrodes attached to opposite ends of the current generating circuit; the names "source" and "return" are used herein for clarity to differentiate where the electrode is placed with respect to the implantable device, and to clarify the meaning of "positive" and "negative" current in a system for applying transcutaneous current. The source electrode 114 may be the electrode placed above the implantation site of a receiving electrode of the implantable device, and the return electrode 116 may be the electrode placed at a different location (e.g., above or near the implantation site of the output electrodes of the implantable device). Positive current, as used herein, refers to current passing from the source electrode (e.g., the electrode proximate the receiving electrode) to the return electrode (e.g., the electrode placed at a different location), and negative current, as used herein, refers to current passing from the return electrode to the source electrode. In some aspects, the electrodes of the external generator 110 may be interchangeable and whichever electrode is placed above the receiving electrode of the implantable device may be referred to as the source electrode as used herein.

The implantable device 130 includes a receiver 134, an output electrode 136, and a lead 132 between the receiver 134 and the output electrode 136. The receiver 134 may be a sealed enclosure, such as a hermetically sealed enclosure. The receiver 134 may include a receiving electrode 133 exposed to the surrounding tissue. For example, the receiver housing may include a conductive material which may function as a receiving electrode. The receiving electrode and the output electrode 136 are exposed, allowing them to contact the tissue at their respective implantation sites. The receiving electrode is electrically connected to the output electrode 136 through the electronics of the receiver and an electrical conductor inside the lead 132.

In some aspects, the output electrode 136 may be a stimulating electrode. It may be implanted at a location targeted for delivery of stimulation by the system for applying transcutaneous current. The current passing through the output electrode 136 may stimulate the tissue surrounding the output electrode 136 (e.g., may activate a nerve at the implantation site of the output electrode 136).

The external generator 110 may apply transcutaneous current between the source electrode 114 and the return electrode 116. For example, the external generator 110 may apply bursts of alternating transcutaneous current between the source electrode 114 and the return electrode 116. Some of the applied transcutaneous current may pass directly between the source electrode 114 and the return electrode 116 through the tissue of the subject. The rest of the applied transcutaneous current may pass from the source electrode 114 along a first implant tissue path 117 to the receiving electrode 113 of the implantable device 130, through the implantable device 130, and from the output electrode 136 along a second implant tissue path 118 to the return electrode 116.

The implantable device 130 may include a rectification circuit. For example, the rectification circuit may be in the receiver 134. The rectification circuit in the implantable device 130 allows current to pass from the receiving electrode at the receiver 134 to the output electrode 136 but inhibits or entirely prevents current flow from the output electrode 136 to the receiving electrode. Accordingly, the current 160 passing through the implantable device 130 may include the positive portions of the transcutaneous current applied by the external generator 110 but may not include negative portions of the transcutaneous current applied by the external generator 110.

Alternatively, the rectification circuit in the implantable device 130 may be oriented to allow current to pass from the output electrode 136 to the receiving electrode but to inhibit or entirely prevent current flowing from the receiving electrode to the output electrode 136. In that configuration, the current passing through the implantable device 130 may include the negative portions of the transcutaneous current applied by the external generator 110, but may not include the positive portions of the transcutaneous current applied by the external generator 110.

In some aspects, the external generator 110 may be able to determine the functionality of the implantable device 130. The functionality of the implantable device may refer both to the implantable device 130 operating according to the expected operation of the rectification circuit for the implantable device 130 and to the implantable device 130 coupling with the external generator 110 allowing the portion of transcutaneous current from the external generator 110 to pass through the implantable device 130. The external generator 110 may determine the functionality of the implantable device 130 without having any measurement circuits or any other special circuits in the implantable device.

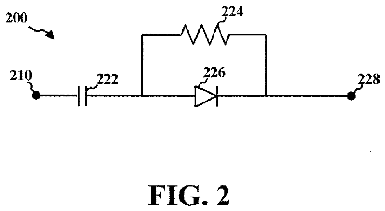

FIG. 2 is a diagram illustrating an embodiment of a rectification circuit 200 for the implantable device 130 described with respect to FIG. 1. The rectification circuit 200 is connected between the receiving electrode 210 and the output electrode 228. The rectification circuit 200 includes a capacitor 222, a resistor 224, and a diode 226. In some aspects, the rectification circuit 200 may include additional electronic components, such as electrostatic discharge protection components.

The rectification circuit 200 is configured for anodic stimulation. The diode 226 is oriented to allow current to pass from the receiving electrode 210 to the output electrode 228. The diode 226 may be a Schottky diode.

The capacitor 222 is connected between the receiving electrode 210 and the diode 226, serving as a DC block to ensure that the stimulation delivered to the nerve through the implantable device is charge balanced. The capacitor 222 may have a capacitance suitable to allow for the high frequency rectified current pulse to pass through with minimal attenuation or distortion, while allowing for the capacitor to adequately or fully discharge during the discharge periods between stimulation periods. For example, the capacitance of the capacitor 222 may be on the order of several micro-Farads.

The resistor 224 is connected in parallel with the diode 226. The resistor 224 may provide a path for the capacitor 222 to discharge during the discharge periods. The resistor 224 may have a resistance suitable to present high impedance relative to the diode 226, such that little or no current flows through the resistor 224 during the stimulation periods, but to allow for the capacitor 222 to adequately discharge during the discharge periods. For example, the resistance of the resistor 224 may be on the order of several kilo-Ohms.

In some aspects, the rectification circuit 200 may contain additional components, such as component to protect against electro-static discharge (ESD). In some aspects, the rectification circuit 200 may include fewer components, for example, the rectification circuit 200 may contain only the rectification diode 226. In some aspects, the rectification circuit 200 may contain rectification components other than a diode, for example a transistor.

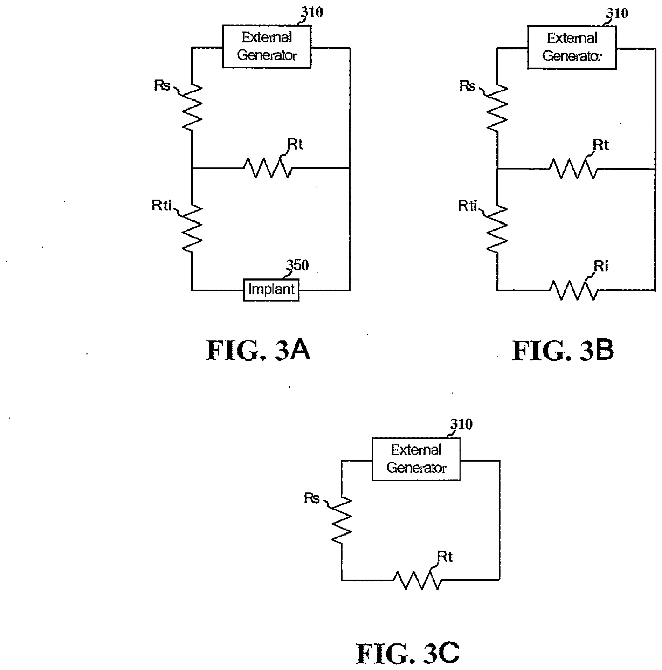

FIG. 3A is a diagram illustrating an electrical model representing a system for applying transcutaneous current in-vivo. The model includes the external generator 310, a first resistor Rs, a second resistor Rt, a third resistor Rti, and an implantable device 350. The external generator 310 may be the external generator 110 described above with respect to FIG. 1. The implantable device 350 may be the implantable device 130 described above with respect to FIG. 1.

The first resistor Rs represents the combined impedances of the source electrode and the return electrode of the external generator 310 and of the skin beneath the source electrode and the return electrode, which may be referred to hereinafter as the electrode-skin impedance. The second resistor Rt represents the impedance of the electrical path through the tissue for current passing between the source electrode and the return electrode without passing through the implantable device 350, which may be referred to hereinafter as the tissue path impedance. The third resistor Rti represents the impedance of the electrical path through the tissue for current passing through the implantable device 350.

The implantable device 350 includes a component with different forward and backward impedances in the path of current passing through the implantable device 350. For example, the implantable device 350 may include a diode rectifying current passing through the implantable device 350, as described above with respect to the diode 226 of FIG. 2. The impedance of the implantable device 350 may be different when the external generator 310 applies positive current than when the external generator 310 applies negative current.

FIG. 3B is a diagram illustrating an electrical model representing the system for applying transcutaneous current while the implanted component with different forward and backward impedances is conducting. For example, the external generator 310 may apply positive current to the skin of the subject and a diode in the current path of the implantable device 350 may be oriented to allow positive current to pass through the channel. The impedance of the implant 350 is represented by a fourth resistor Ri. When the external generator 310 applies the positive current to the skin of the subject, some of the current passes through the second resistor Rt representing the tissue path impedance and the rest of the current passes through the fourth resistor Ri representing the implant impedance.

FIG. 3C is a diagram illustrating an electrical model representing the system for applying transcutaneous current while the implanted component with different forward and backward impedances is not conducting. For example, the external generator 310 may apply negative current to the skin of the subject and the diode in the current path of the implantable device 350 may be oriented to allow positive current to pass. Little to no current may pass through the implantable device 350, so the implantable device 350 is represented as an open circuit. When the external generator 310 applies the negative current to the skin of the subject, all of the current passes through the second resistor 330 representing the tissue path impedance.

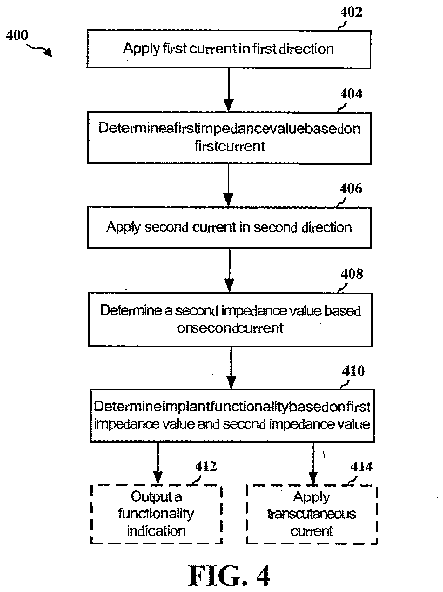

FIG. 4 is a flowchart 400 illustrating a method of detecting functionality in an implantable device. In some aspects, the method may be performed by an external generator (e.g., the external generator 110 of FIG. 1).

At 402, the external generator applies a first current in a first direction to the skin of a subject. An implantable device (e.g., the implantable device 130 of FIG. 1) may be implanted beneath the skin of the subject to present a path for current from the external generator. The implantable device may include a component with a different forward and backward impedance in the path of the current (e.g., a diode). The first current may be in the direction for which the component with a different forward and backward impedance has a low impedance. For example, the component may be a diode and the first current may be in the direction that the diode conducts.

At 404, the external generator determines a first impedance value based on the first current. The first impedance value may be or may correspond to the impedance between the source electrode and the return electrode when the external generator is applying the first current in the first direction. In some aspects, the external generator may measure the impedance between the source electrode and the return electrode while the first current is being applied to determine the first impedance value. In some aspects, the external generator may measure the voltage or the current between the source electrode and the return electrode while the first current is being applied to determine the first impedance value. In some aspects, the external generator may receive the first impedance value from another device which measured the impedance, voltage, or current between the source electrode and the return electrode when the first current was being applied.

At 406, the external generator applies a second current in a second direction to the skin of the subject. The second current may be in the opposite direction of the first current (e.g., the first current may be positive and the second current may be negative, or the first current may be negative and the second current may be positive). The second current may be in the direction for which the component with a different forward and backward impedance has a high impedance. For example, the component may be a diode and the second current may be in the direction that the diode does not conduct.

At 408, the external generator determines a second impedance value based on the second current. The second impedance value may be or may correspond to the impedance between the source electrode and the return electrode when the external generator is applying the second current in the second direction. In some aspects, the external generator may measure the impedance between the source electrode and the return electrode while the second current is being applied to determine the firs second t impedance value. In some aspects, the external generator may measure the voltage or the current between the source electrode and the return electrode while the second current is being applied to determine the second impedance value. In some aspects, the external generator may receive the second impedance value from another device which measured the impedance, voltage, or current between the source electrode and the return electrode when the second current was being applied.

At 410, the external generator determines implantable device functionality based on the first impedance value determined at 404 and the second impedance value determined at 408. The external generator may determine whether the difference between the first impedance value and the second impedance value exceeds a threshold value. In some aspects, the threshold value may be whether the difference between the first impedance value and the second impedance value is greater than 1%. In some aspects, the threshold value may be whether the difference between the first impedance value and the second impedance value is greater than 5%. In some aspects, the threshold value may be whether the difference between the first impedance value and the second impedance value is greater than 10%. In some aspects, other threshold values may be used. If the difference between the first impedance value and the second impedance value exceeds the threshold value, the external generator may determine that the implantable device is functional. If the difference between the first impedance value and the second impedance value does not exceed the threshold value, the external generator may determine that the implantable device is not functional. The implantable device may not be working as expected, or the electrodes of the external generator may on the skin of the subject not be aligned with the implantable device.

In some aspects, the external generator may determine implantable device functionality based on the first impedance value and the second impedance value by transmitting the first impedance value and the second impedance value to an external device (e.g., a remote control or a smart phone) and receiving the implantable device functionality from the external device.

In some aspects, at 412, the external generator may output a functionality indication based on the implant functionality determined at 410. The external generator may utilize a functionality indication component to output the functionality indication. For example, the functionality indication component may be an indicator light on the external generator and outputting the functionality indication may be lighting or not lighting the indicator light. The functionality indication component may be a display of a user interface and outputting the functionality indication may be displaying or not displaying an element on the display. The functionality indication component may be a speaker and outputting the functionality indication may be utilizing the speaker to make a sound or not make a sound. In some aspects, the external generator may output the functionality indication by transmitting the functionality indication to an external device such as a remote control or a smart phone in communication with the external generator. If the external generator determined that the implantable device is functional, the external generator may output a functionality indication identifying that the implantable device is functional. If the external generator determined that the implantable device is not functional, the external generator may output a functionality indication identifying that the implantable device is not functional.

In some aspects, 410 and 412 may not be distinct actions, and determining implant functionality may be outputting the functionality indication. For example, at 410, the external generator may output a functionality indication identifying that the implantable device is functional based on the difference between the first impedance value and the second impedance value exceeding the threshold value or may output a functionality indication identifying that the implantable device is not functional based on the difference between the first impedance value and the second impedance value not exceeding the threshold value.

In some aspects, at 414, the external generator may apply transcutaneous current based on the implant functionality determined at 410. In some aspects, the external generator may apply therapeutic transcutaneous current based on determining that the implantable device is functional at 410 and may not apply therapeutic transcutaneous current based on determining that the implantable device is not functional at 410. In some aspects, the external generator may apply current at a first amplitude at 402 and 406. The external generator may increase the amplitude of applied transcutaneous current based on determining that the implantable device is functional at 410. The external generator may continue to apply transcutaneous current at the first amplitude (and continue determining impedance values based on that transcutaneous current) based on determining that the implantable device is not functional at 410. In some aspects, the external generator may increase the amplitude of applied transcutaneous current based on determining that the implantable device is not functional at 410. The implantable device may include a diode with a non-linear voltage current characteristic. The first current and/or the second current may not have resulted in high enough implant current to forward bias the diode, preventing the external generator from detecting a difference between the first impedance value and the second impedance value at 410, but increasing the amplitude of the current applied may result in an implant current high enough to do so.

In some aspects, 410 and 414 may not be distinct actions, and determining implant functionality may be applying transcutaneous current. For example, at 410, the external generator may apply or not apply therapeutic transcutaneous current based on whether the difference between the first impedance value and the second impedance value exceeds the threshold value, or may increase the amplitude of the applied transcutaneous current based on whether the difference between the first impedance value and the second impedance value exceeds the threshold value.

In some aspects, the external generator may apply alternating transcutaneous current to the skin of a subject to deliver the current to the implant and generate a therapeutic effect in the subject. In some aspects, the alternating transcutaneous current may be bursts of alternating transcutaneous current. The first current described above may be one half-cycle of the alternating transcutaneous current (e.g., a positive half-cycle) and the second current described above may be one half-cycle of the alternating transcutaneous current having the opposite polarity (e.g., a negative half-cycle). The external generator may apply the alternating transcutaneous current to generate the therapeutic effect and may measure the impedances and determine implant functionality based on the same alternating transcutaneous current.

The method above has been described above as being performed by the external generator. In some aspects, some or all of the method may be performed by a separate device. For example, the external generator may apply the first current at 402 and apply the second current at 406, and the separate device may determine the first impedance value at 404, may determine the second impedance value at 408, and may determine the implant functionality at 410.

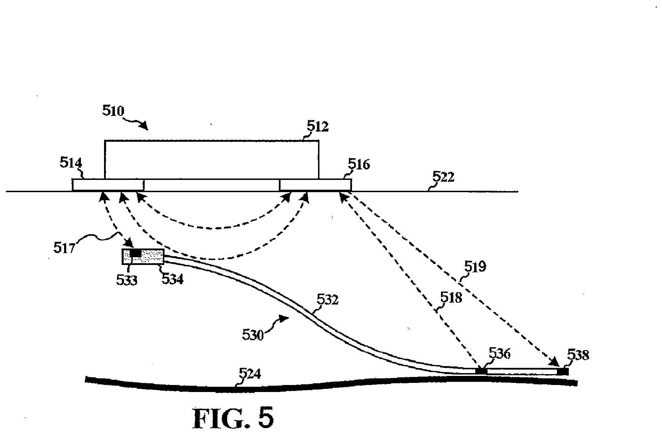

FIG. 5 is a diagram illustrating a multi-channel system for applying transcutaneous current. The system includes an external generator 510 and an implantable device 530. The external generator 510 includes a device housing 512, a source electrode 514, and a return electrode 516. The external generator 510 may apply current between the source electrode 514 and the return electrode 516. For example, the external generator 510 may be an external generator that applies electrical current between the source electrode 514 and the return electrode 516. The source electrode 514 and the return electrode 516 may be attached directly to the device housing 512, or one or both may be attached to the device housing 512 by a lead.

The source electrode 514 and the return electrode 516 may be similar or identical electrodes attached to opposite ends of the current generating circuit; the names "source" and "return" are used herein for clarity to differentiate where the electrode is placed with respect to the implantable device, and to clarify the meaning of "positive" and "negative" current in a system for applying transcutaneous current. The source electrode 514 may be the electrode placed above the implantation site of a receiving electrode of the implantable device, and the return electrode 516 may be the electrode placed at a different location (e.g., above or near the implantation site of the output electrodes of the implantable device).

The implantable device 530 includes a receiver 534, a receiving electrode 533, a first output electrode 536, a second output electrode 538, and a lead 532. In some aspects, the first output electrode 536 and the second output electrode 538 may be stimulating electrodes. They may be implanted at locations targeted for delivery of stimulation by the system for applying transcutaneous current. The current passing through the first output electrode 536 and the second output electrode 538 may stimulate the tissue surrounding the electrodes (e.g., may activate a nerve at the implantation site of the electrode). In some aspects, the receiving electrode 533 (e.g., with the receiver 534) may be at or near one end of the lead 532, and the first output electrode 536 and the second output electrode 538 may be at the other end of the lead 532. The receiving electrode 533, the first output electrode 536, and the second output electrode 538 are exposed, allowing them to contact the tissue at their respective implantation sites, and may be connected by insulated conductors running inside the implantable device 530, in particular via the implanted lead 532.

The implantable device 530 includes a first channel and a second channel. The first and second channels may be separate electrical paths for delivering electrical current through the implantable device. The first channel connects the receiving electrode 533 to the first output electrode 536 and may allow current to pass between the receiving electrode 533 and the first output electrode 536. The second channel connects the receiving electrode 533 to the second output electrode 538 and may allow current to pass between the receiving electrode 533 and the second output electrode 538.

The implantable device 530 may include a first rectification element and a second rectification element. The first channel may include the first rectification element and the second channel may include the second rectification element. The first rectification element may be electrically connected to the receiving electrode 533 and the first output electrode 536, and may be configured to allow current to pass from the receiving electrode 533 to the first output electrode 536, and to inhibit current from flowing from the first output electrode 536 to the receiving electrode 533 (e.g., may be configured for anodic stimulation). The second rectification element may be electrically connected to the receiving electrode 533 and the second output electrode 538, and may be configured to allow current to pass from the second output electrode 538 to the receiving electrode 533, and to inhibit current from flowing from the receiving electrode 533 to the second output electrode 538 (e.g., may be configured for cathodic stimulation).

The receiving electrode 533 is electrically connected to the first output electrode 536 through the first receiving circuit and an insulated conductor inside the lead 532. The receiving electrode 533 is electrically connected to the second output electrode 538 through the second receiving circuit and a second insulated conductor inside the lead 532.

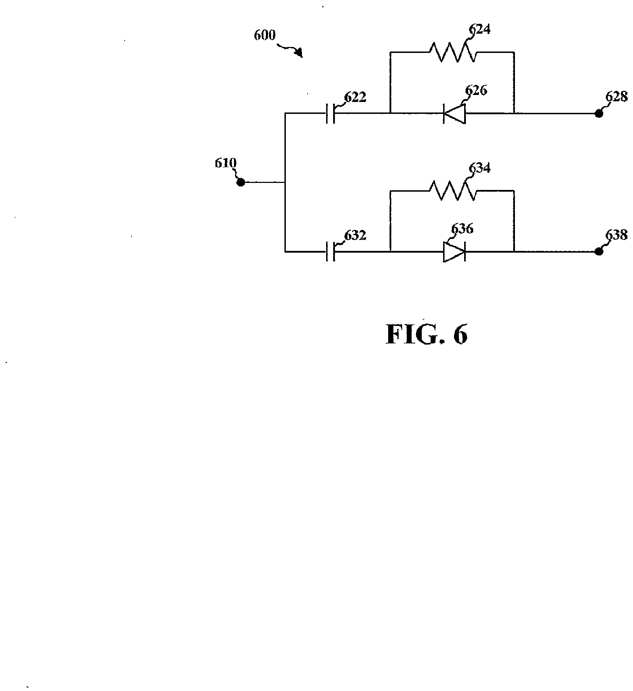

FIG. 6 is a circuit diagram of an implantable device 600 for applying multi-channel stimulation. For example, the implantable device 600 may be the implantable device 530 described above with respect to FIG. 5. The implantable device 600 includes a receiving electrode 610, a first output electrode 638, and a second output electrode 628.

The implantable device 600 includes a first channel connecting the receiving electrode 610 with the first output electrode 638. The first channel includes a first diode 636, a first capacitor 632, and a first resistor 634. The implantable device 600 includes a second channel connecting the receiving electrode 610 with the second output electrode 628. The second channel includes a second diode 626, a second capacitor 622, and a second resistor 624.

The anode of the first diode 636 is coupled to the receiving electrode 610 and the cathode of the first diode 636 is coupled to the first output electrode 638. The first diode 636 will present a low impedance to current flowing from the receiving electrode 610 to the first output electrode 638 in the first channel. The first diode 636 will present a high impedance to current flowing from the first output electrode 638 to the receiving electrode 610.

The cathode of the second diode 626 is coupled to the receiving electrode 610 and the anode of the second diode 626 is coupled to the second output electrode 628. The second diode 626 will present a high impedance to current flowing from the receiving electrode 610 to the second output electrode 628 in the second channel. The second diode 626 will present a low impedance to current flowing from the second output electrode 628 to the receiving electrode 610.

In some aspects, the first diode 636 and/or the second diode 626 may be a Schottky diode.

The first capacitor 622 is connected between the receiving electrode 610 and the first diode 636. The second capacitor 632 is connected between the receiving electrode 610 and the second diode 624. The first capacitor 632 and the second capacitor 622 may serve a DC blocking function to ensure that the stimulation delivered to the nerve through their respective channels is charge balanced. The first capacitor 632 and the second capacitor 622 may have capacitances suitable to allow for the high frequency rectified current pulse to pass through with minimal attenuation or distortion, while allowing for the first capacitor 632 and the second capacitor 622 to adequately or fully discharge during the discharge periods between stimulation periods. For example, the capacitance of the first capacitor 632 and/or the second capacitor 622 may be on the order of several micro-Farads.

The first resistor 634 is connected in parallel with the first diode 636. The second resistor 634 is connected in parallel with the second diode 636. The first resistor 634 and the second resistor 624 may provide a path for the first capacitor 632 and the second capacitor 622 to discharge during the discharge periods. The first resistor 634 and the second resistor 624 may have resistances suitable to present high impedance relative to the first diode 636 and the second diode 626, such that little or no current flows through the first resistor 634 and the second resistor 624 during the stimulation periods, but to allow for the first capacitor 632 and the second capacitor 622 to adequately discharge during the discharge periods. For example, the resistance of the first resistor 634 and/or the second resistor 624 may be on the order of several kilo-Ohms.

When an external generator applies alternating transcutaneous current to the tissue surrounding the implantable device 600, the first diode 636 and the second diode 626 rectify the transcutaneous current. Positive portions of the alternating transcutaneous current may pass through the first channel and negative portions of the alternating transcutaneous current may pass through the second channel.

Note that although both channels are shown to be connected to the same receiving electrode 610, in some aspects each channel can be connected to a different receiving electrode. In some aspects, rectification circuits may include additional components, such as ESD protection components. In some aspects, rectification circuits may include fewer components, for example the rectification circuit may include only a diode. In some aspects, rectification circuits may utilize a rectification component other than a diode, such as a transistor.

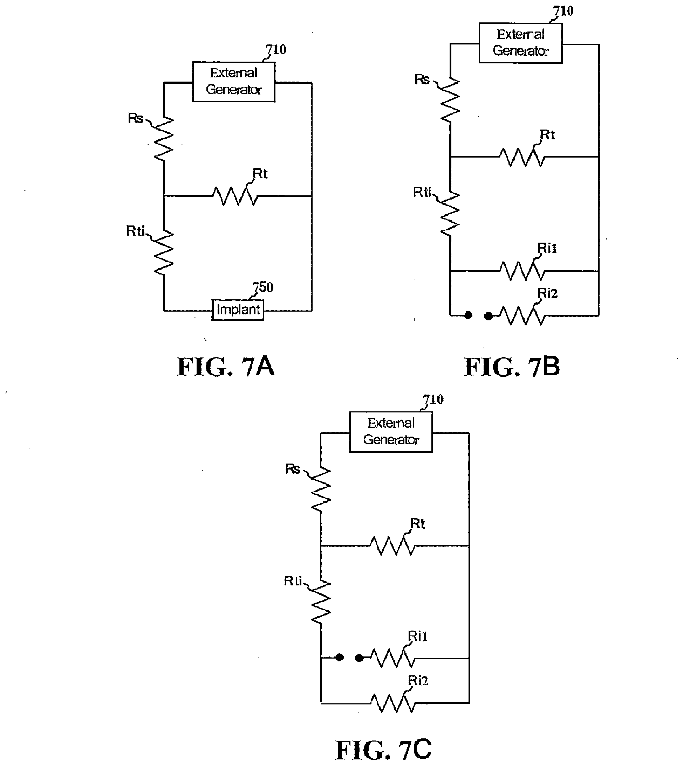

FIG. 7A is a diagram illustrating an electrical model representing a multi-channel system for applying transcutaneous current in-vivo. The model includes the external generator 710, a first resistor Rs, a second resistor Rt, a third resistor Rti, and a multi-channel implantable device 750. The external generator 710 may be the external generator 510 described above with respect to FIG. 5. The multi-channel implantable device 750 may be the implantable device 530 described above with respect to FIG. 5.

The first resistor Rs represents the combined impedances of the source electrode and the return electrode of the external generator 710 and of the skin beneath the source electrode and the return electrode, which may be referred to hereinafter as the electrode-skin impedance. The second resistor Rt represents the impedance of the electrical path through the tissue for current passing between the source electrode and the return electrode without passing through the multi-channel implantable device 750, which may be referred to hereinafter as the tissue path impedance. The third resistor Rti represents the impedance of the electrical path through the tissue for current passing through the multi-channel implantable device 750.

The multi-channel implantable device 750 includes a first component with different forward and backward impedances in the first channel of the implantable device 750 and includes a second component with different forward and backward impedances in the second channel of the implantable device 750. The first component and the second component may have high impedances in opposite directions. For example, the first channel of the implantable device 750 may include a diode rectifying current in the positive direction as described above with respect to the diode 636 of FIG. 6, and the second channel of the implantable device 750 may include a diode rectifying current in the negative direction as described above with respect to the diode 626 of FIG. 6.

FIG. 7B is a diagram illustrating an electrical model representing the system for applying transcutaneous current to the implantable device while the first component of the first channel is conducting and the second component of the second channel is not conducting. A fourth resistor Ri1 represents the impedance of the first channel of the implantable device 750 and a fifth resistor Ri2 represents the impedance of the second channel of the implantable device 750. As the second component in the second channel is not conducting, the impedance of the fifth resistor Ri2 may be high enough that little to no current passes through the fifth resistor Ri2 (represented as an open circuit in the path of the fifth resistor Ri2). Most or all of the current applied by the external generator 710 may pass through the second resistor Rt and the fourth resistor Ri1.

FIG. 7C is a diagram illustrating an electrical model representing the system for applying transcutaneous current to the implantable device while the second component of the second channel is conducting and the first component of the first channel is not conducting. As the first component in the first channel is not conducting, the impedance of the fourth resistor Ri1 may be high enough that little to no current passes through the fourth resistor Ri1 (represented as an open circuit in the path of the fourth resistor Ri1. Most or all of the current applied by the external generator 710 may pass through the second resistor Rt and the fifth resistor Ri2.

The first path of the implantable device 750 and the second path of the implantable device 750 may have the same or may have similar impedances. The impedances between the electrodes of the external generator 750 may therefore be the same or similar during the application of positive current and during the application of negative current where the positive current and the negative current have similar characteristics but opposite polarity.

In some aspects, the implantable device 750 may include components with a non-linear current voltage response in the current path of the first channel and the current path of the second channel. For example, the components with non-linear current voltage responses may be the rectifying diodes included in the first channel and the second channel. When the external generator 710 applies transcutaneous current (e.g., a single cycle or brief pulse of alternating transcutaneous current) to the skin of the subject, if the resulting voltage across the diodes is high enough to forward bias one of the diodes, that diode will conduct. During application of current in one direction, the current applied by the external generator 750 will pass through the second resistor Rt and the fourth resistor Ri1. During application of the current in the opposite direction, the current applied by the external generator 750 will pass through the second resistor Rt and the fifth resistor Ri2. As the fourth resistor Ri1 and the fifth resistor Ri2 have the same or similar impedances, the impedance between the electrodes of the external generator 750 will be similar during both positive and negative cycles.

When the external generator 710 applies transcutaneous current (e.g., a single cycle or brief pulse of alternating transcutaneous current) to the skin of the subject, if the resulting voltage across the diodes is not high enough to forward bias the diodes, neither diode will conduct. The impedances of the fourth resistor Ri1 and the fifth resistor Ri2 may be high enough that little to no current passes through them. Most or all of the current applied by the external generator 710 may pass through the second resistor Rt.

In some aspects, the implantable device 750 may include capacitors in the current path of the first channel and in the current path of the second channel. For example, the capacitor in the current path of the first channel may be the capacitor 632 of FIG. 6 and the capacitor in the current path of the second channel may be the capacitor 622 of FIG. 6. As the capacitors are exposed to current over time, they will charge and their impedance will increase. When the external generator 710 applies a brief burst of alternating transcutaneous current to the skin of the subject, the capacitors may experience little or no increase in impedance. During application of current in one direction, the current applied by the external generator 750 will pass through the second resistor Rt and the fourth resistor Ri1. During application of the current in the opposite direction, the current applied by the external generator 750 will pass through the second resistor Rt and the fifth resistor Ri2. As the fourth resistor Ri1 and the fifth resistor Ri2 have the same or similar impedances, the impedance between the electrodes of the external generator 750 will be similar during both positive and negative cycles.

When the external generator 710 applies alternating transcutaneous current over a long enough period that the capacitors in the first channel and the second channel charge (e.g., applies a continuous wave of alternating transcutaneous current), the capacitors will have a high impedance. The impedances of the fourth resistor Ri1 and the fifth resistor Ri2 that represent the impedances of the first channel and the second channel may be high enough that little or no current passes through them. Most or all of the current applied by the external generator 710 may pass through the second resistor Rt.

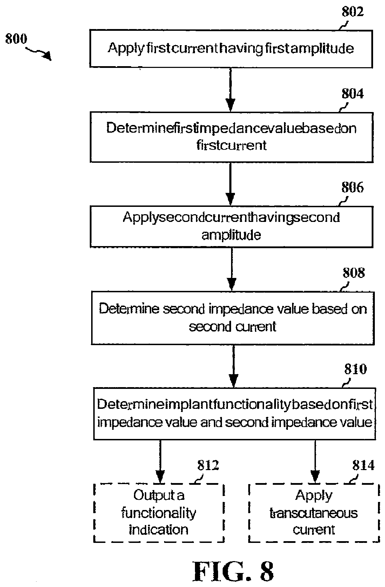

FIG. 8 is a flowchart 800 illustrating a method of detecting functionality in a multi-channel implantable device. In some aspects, the method may be performed by an external generator (e.g., the external generator 510 of FIG. 5).

An implantable device (e.g., the implantable device 530 of FIG. 5) may be implanted beneath the skin of the subject to present a path for current from the external generator. The implantable device may include a first channel and a second channel. The first channel may include a first diode which may rectify current to allow current from the external generator to pass in the positive direction. The second channel may include a second diode which may rectify current to allow current from the external generator to pass in the negative direction.

If the implantable device is functional, the impedance measured in the first direction should be similar to the impedance measured in the opposite direction. If the difference between the two impedances exceeds certain threshold, the implantable device may be considered not functional, and corresponding indication may be presented to a user.

At 802, the external generator applies a first current to the skin of a subject having a first amplitude. The first current may be alternating transcutaneous current. The first amplitude of the first current is low enough that it does not result in the first diode or the second diode being forward biased during application of the first current. In some aspects, the first amplitude may be between .1 mA and 10 mA. In some aspects, the first amplitude may be between .9 mA and 1.1 mA. In some aspects, the first amplitude may be 1 mA. In some aspects, the first amplitude of the first current may have other values.

Depending on the approach for determining the functionality of the implant, different values of transcutaneous current may be applied. Some considerations for selection of the current amplitude or current duration values are presented below. It should also be noted that different values of the applied current or voltage may produce not only different technical effect, but also cause a physiological effect. Technical effect is, for example, causing different charge of a capacitor in the implantable device and a different effective impedance of the capacitor or having a different impedance of the rectifying diode depending on the direction and the amplitude of the applied current or voltage.

A physiological effect is when the applied current causes tissue activation, for example, at the targeted location of the implant and/or under the surface electrodes of the external generator. Since tissue activation may be not desired, the external generator may perform measurement of the impedances and assessment of implant functionality infrequently. For example, the external generator may perform assessment of implant functionality every several hours or once per day. In some aspects, the external generator may apply only a single pulse or a few pulses of current when assessing the implant functionality. Applying single or few pulses may not cause undesired sensation or other response by the subject.

Some of the approaches proposed in this disclosure use measurements in the different location along the current-voltage characteristic of the implanted diode. For example, if it is desired to measure the impedance when the diode presents low impedance, the current flowing through the diode in the forward direction needs to be of the order of several mA. The pick-up ratio of the implantable device (e.g., the ratio of the current flowing via the implantable device to the total transcutaneous current applied by the external generator) may be on the order of 10% - 20%. Therefore, in order to deliver several mA to the diode, the transcutaneous current may be of the order of several tens of mA. For example, if the diode current is 3mA, and the pick-up ratio is 10%, the signal generator may deliver current of 30mA.

If it is desired to measure the impedance when the diode presents high impedance, the diode current may be very low, on the order of a fraction of a mA. In this case the external generator needs to deliver current on the order of several mA or less.

At 804, the external generator determines a first impedance value based on the first current. The first impedance value may be or may correspond to the impedance between the source electrode and the return electrode when the external generator is applying the first current. In some aspects, the external generator may measure the impedance between the source electrode and the return electrode while the first current is being applied to determine the first impedance value. In some aspects, the external generator may measure the voltage or the current between the source electrode and the return electrode while the first current is being applied to determine the first impedance value. In some aspects, the external generator may receive the first impedance value from another device which measured the impedance, voltage, or current between the source electrode and the return electrode when the first current was being applied.

At 806, the external generator applies a second current to the skin of the subject having a second amplitude. The second current may be alternating transcutaneous current. The second amplitude of the second current may be high enough that it forward biases the first diode in the first channel during positive portions of the second current and forward biases the second diode in the second channel during negative portions of the second current. In some aspects, the second amplitude may be between 5 mA and 500 mA. In some aspects, the second amplitude may be between 40 mA and 60 mA. In some aspects, the second amplitude may be 50 mA.

At 808, the external generator determines a second impedance value based on the second current. The second impedance value may be or may correspond to the impedance between the source electrode and the return electrode when the external generator is applying the second current in the second direction. In some aspects, the external generator may measure the impedance between the source electrode and the return electrode while the second current is being applied to determine the second impedance value. In some aspects, the external generator may measure the voltage or the current between the source electrode and the return electrode while the second current is being applied to determine the second impedance value. In some aspects, the external generator may receive the second impedance value from another device which measured the impedance, voltage, or current between the source electrode and the return electrode when the second current was being applied.

At 810, the external generator determines implantable device functionality based on the first impedance value determined at 804 and the second impedance value determined at 808. In some aspects, the external generator may determine whether the difference between the first impedance value and the second impedance value exceeds a first threshold value. If the implantable device is functional and current from the external generator is passing through the implantable device, the first impedance value determined during the first current, when the diodes are not conducting, should be higher than the second impedance value determined during the second current, when the diodes are conducting. If the difference between the first impedance value and the second impedance value exceeds the first threshold value, the external generator may determine that the implantable device is functional. If the difference between the first impedance value and the second impedance value does not exceed the first threshold value, the external generator may determine that the implantable device is not functional. The implantable device may not be working as expected, or the electrodes of the external generator may on the skin of the subject not be aligned with the implantable device. In some aspects, the first threshold value may be whether the difference between the first impedance value and the second impedance value is greater than 1%. In some aspects, the first threshold value may be whether the difference between the first impedance value and the second impedance value is greater than 5%. In some aspects, the first threshold value may be whether the difference between the first impedance value and the second impedance value is greater than 10%.

In some aspects, the external generator may determine whether the difference between the first impedance value and the second impedance value exceeds a second threshold value. If one of the channels of the implantable device is functional and the current from the external generator is passing through the implantable device, but the other channel is not functional, there may be a difference in impedance between the impedance of the first channel and the impedance of the second channel. Therefore, the difference between the first impedance value and the second impedance value may be higher than it would be if both of the channels of the implantable device were functioning. If the difference between the first impedance value and the second impedance value does not exceed the second threshold value, the external generator may determine that the implantable device is functional. If the difference between the first impedance value and the second impedance value exceeds the second threshold value, the external generator may determine that the implantable device is not functional. In some aspects, the second threshold value may be whether the difference between the first impedance value and the second impedance value is greater than 1%. In some aspects, the second threshold value may be whether the difference between the first impedance value and the second impedance value is greater than 5%. In some aspects, the second threshold value may be whether the difference between the first impedance value and the second impedance value is greater than 10%.

In some aspects, the external generator may determine whether the difference between the first impedance value and the second impedance value exceeds the first threshold value and exceeds the second threshold value. If the difference between the first impedance value and the second impedance value exceeds the first threshold value and does not exceed the second threshold value, the external generator may determine that the implantable device is functional. If the difference between the first impedance value and the second impedance value does not exceed the first threshold value or does exceed the second threshold value, the external generator may determine that the implantable device is not functional.

In some aspects, the external generator may determine implantable device functionality based on the first impedance value and the second impedance value by transmitting the first impedance value and the second impedance value to an external device (e.g., a remote control or a smart phone) and receiving the implantable device functionality from the external device.

In some aspects, at 812, the external generator may output a functionality indication based on the implant functionality determined at 810. The external generator may utilize a functionality indication component to output the functionality indication. For example, the functionality indication component may be an indicator light on the external generator and outputting the functionality indication may be lighting or not lighting the indicator light. The functionality indication component may be a display of a user interface and outputting the functionality indication may be displaying or not displaying an element on the display. The functionality indication component may be a speaker and outputting the functionality indication may be utilizing the speaker to make a sound or not make a sound. In some aspects, the external generator may output the functionality indication by transmitting the functionality indication to an external device such as a remote control or a smart phone in communication with the external generator. If the external generator determined that the implantable device is functional, the external generator may output a functionality indication identifying that the implantable device is functional. If the external generator determined that the implantable device is not functional, the external generator may output a functionality indication identifying that the implantable device is not functional.

In some aspects, 810 and 812 may not be distinct actions, and determining implant functionality may be outputting the functionality indication. For example, at 810, the external generator may output a functionality indication identifying that the implantable device is functional based on the difference between the first impedance value and the second impedance value exceeding the first threshold value or may output a functionality indication identifying that the implantable device is not functional based on the difference between the first impedance value and the second impedance value not exceeding the first threshold value.

In some aspects, at 814, the external generator may apply transcutaneous current based on the implant functionality determined at 810. In some aspects, the external generator may apply therapeutic transcutaneous current based on determining that the implantable device is functional at 810 and may not apply therapeutic transcutaneous current based on determining that the implantable device is not functional at 810. In some aspects, the external generator may apply current at a third amplitude based on determining that the implantable device is functional at 810. The external generator may continue to apply transcutaneous current at the first amplitude and the second amplitude (and continue measuring impedances based on that transcutaneous current) based on determining that the implantable device is not functional at 810.

In some aspects, 810 and 814 may not be distinct actions, and determining implant functionality may be applying transcutaneous current. For example, at 810, the external generator may apply or not apply therapeutic transcutaneous current based on whether the difference between the first impedance value and the second impedance value exceeds the first threshold value or the second threshold value, or may apply transcutaneous current at the third amplitude based on whether the difference between the first impedance value and the second impedance value exceeds the first threshold value or the second threshold value.

The method above has been described above as being performed by the external generator. In some aspects, the method may be performed by a separate device.

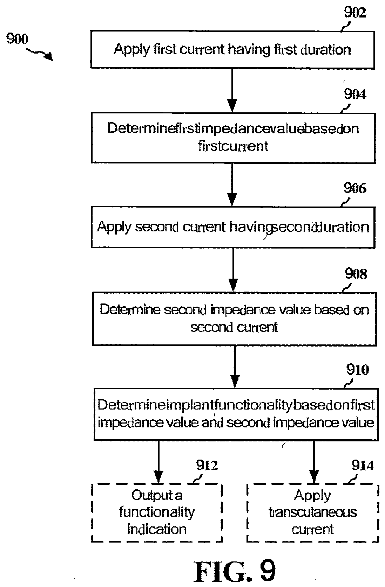

FIG. 9 is a flowchart 900 illustrating a method of determining functionality in a multi-channel implantable device. In some aspects, the method may be performed by an external generator (e.g., the external generator 510 of FIG. 5).

An implantable device (e.g., the implantable device 530 of FIG. 5) may be implanted beneath the skin of the subj ect to present a path for current from the external generator. The implantable device may include a first channel and a second channel. The first channel may include a first capacitor and the second channel may include a second capacitor.

At 902, the external generator applies a first current to the skin of a subject having a first duration. The first current may be alternating transcutaneous current. In some aspects, the first duration of the first current is short enough that the first current may build little charge on the first capacitor and the second capacitor over the first duration. In some aspects, the first duration of the first current is short enough that the first current does cause the first capacitor and the second capacitor to have effective impedances [greater than 10%] of their impedances at full charge. In some aspects, applying the first current over the first duration may be applying a single cycle of alternating current.

At 904, the external generator determines a first impedance value based on the first current. The first impedance value may be or may correspond to the impedance between the source electrode and the return electrode when the external generator is applying the first current. In some aspects, the external generator may measure the impedance between the source electrode and the return electrode while the first current is being applied to determine the first impedance value. In some aspects, the external generator may measure the voltage or the current between the source electrode and the return electrode while the first current is being applied to determine the first impedance value. In some aspects, the external generator may receive the first impedance value from another device which measured the impedance, voltage, or current between the source electrode and the return electrode when the first current was being applied.

At 906, the external generator applies a second current to the skin of the subject having a second duration. The second current may be alternating transcutaneous current. In some aspects, the second duration of the second current is long enough that the second current may deliver significant charge to the first capacitor and the second capacitor over the second duration. In some aspects, the second duration of the second current is long enough that the second current may charge the first capacitor and the second capacitor to have high effective impedances, or to have effective impedances high enough that they increase the overall impedance of the implant path to exceed a threshold value, as discussed below with respect to 910. For example, the capacitor may be charged, increasing its effective impedance by 10%. In some aspects, the second duration may be greater than one second.

The effective impedance of the capacitor in this context may be defined as an impedance that the capacitor presents to the path of the implant current. The following example illustrates the definition of the effective impedance. Let's consider a simple serial RC circuit connected to the voltage Vg. The effective impedance of the capacitor is Zc = Vc / Ic, where Vc is the voltage across the capacitor and Ic is the current through the capacitor. Simple calculation shows that Zc = Vc·R / (Vg - Vc). The closer the capacitor voltage to Vg, the higher is the effective impedance of the capacitor. Since the capacitor charge is proportional to its voltage (Qc = (1/C)·Vc), the higher is the capacitor charge, the higher is its effective impedance.

At 908, the external generator determines a second impedance value based on the second current. The second impedance value may be or may correspond to the impedance between the source electrode and the return electrode when the external generator is applying the second current in the second direction. In some aspects, the external generator may measure the impedance between the source electrode and the return electrode while the second current is being applied to determine the second impedance value. In some aspects, the external generator may measure the voltage or the current between the source electrode and the return electrode while the second current is being applied to determine the second impedance value. In some aspects, the external generator may receive the second impedance value from another device which measured the impedance, voltage, or current between the source electrode and the return electrode when the second current was being applied.

At 910, the external generator determines implantable device functionality based on the first impedance value determined at 904 and the second impedance value determined at 908. In some aspects, the external generator may determine whether the difference between the first impedance value and the second impedance value exceeds a first threshold value. If the implantable device is functional and current from the external generator is passing through the implantable device, the first impedance value determined during the first current, when the capacitors are not charged and do not have high impedance, should be lower than the second impedance value determined during the second current, when the capacitors are charged and have high impedance. If the difference between the first impedance value and the second impedance value exceeds the first threshold value, the external generator may determine that the implantable device is functional. If the difference between the first impedance value and the second impedance value does not exceed the first threshold value, the external generator may determine that the implantable device is not functional. In some aspects, the first threshold value may be whether the difference between the first impedance value and the second impedance value is greater than 1%. In some aspects, the first threshold value may be whether the difference between the first impedance value and the second impedance value is greater than 5%. In some aspects, the first threshold value may be whether the difference between the first impedance value and the second impedance value is greater than 10%.

In some aspects, the external generator may determine whether the difference between the first impedance value and the second impedance value exceeds a second threshold value. If one of the channels of the implantable device is functional and the current from the external generator is passing through the implantable device, but the other channel is not functional, the difference between the first impedance value and the second impedance value may be higher than it would be if both of the channels of the implantable device were functioning. If the difference between the first impedance value and the second impedance value does not exceed the second threshold value, the external generator may determine that the implantable device is functional. If the difference between the first impedance value and the second impedance value exceeds the second threshold value, the external generator may determine that the implantable device is not functional. In some aspects, the second threshold value may be whether the difference between the first impedance value and the second impedance value is greater than 1%. In some aspects, the second threshold value may be whether the difference between the first impedance value and the second impedance value is greater than 5%. In some aspects, the second threshold value may be whether the difference between the first impedance value and the second impedance value is greater than 10%.

In some aspects, the external generator may determine whether the difference between the first impedance value and the second impedance value exceeds the first threshold value and exceeds the second threshold value. If the difference between the first impedance value and the second impedance value exceeds the first threshold value and does not exceed the second threshold value, the external generator may determine that the implantable device is functional. If the difference between the first impedance value and the second impedance value does not exceed the first threshold value or does exceed the second threshold value, the external generator may determine that the implantable device is not functional.

In some aspects, the external generator may determine implantable device functionality based on the first impedance value and the second impedance value by transmitting the first impedance value and the second impedance value to an external device (e.g., a remote control or a smart phone) and receiving the implantable device functionality from the external device.

In some aspects, at 912, the external generator may output a functionality indication based on the implant functionality determined at 910. The external generator may utilize a functionality indication component to output the functionality indication. For example, the functionality indication component may be an indicator light on the external generator and outputting the functionality indication may be lighting or not lighting the indicator light. The functionality indication component may be a display of a user interface and outputting the functionality indication may be displaying or not displaying an element on the display. The functionality indication component may be a speaker and outputting the functionality indication may be utilizing the speaker to make a sound or not make a sound. In some aspects, the external generator may output the functionality indication by transmitting the functionality indication to an external device such as a remote control or a smart phone in communication with the external generator. If the external generator determined that the implantable device is functional, the external generator may output a functionality indication identifying that the implantable device is functional. If the external generator determined that the implantable device is not functional, the external generator may output a functionality indication identifying that the implantable device is not functional.

In some aspects, 910 and 912 may not be distinct actions, and determining implant functionality may be outputting the functionality indication. For example, at 910, the external generator may output a functionality indication identifying that the implantable device is functional based on the difference between the first impedance value and the second impedance value exceeding the first threshold value or may output a functionality indication identifying that the implantable device is not functional based on the difference between the first impedance value and the second impedance value not exceeding the first threshold value.

In some aspects, at 914, the external generator may apply transcutaneous current based on the implant functionality determined at 910. In some aspects, the external generator may apply therapeutic transcutaneous current based on determining that the implantable device is functional at 910 and may not apply therapeutic transcutaneous current based on determining that the implantable device is not functional at 910.

In some aspects, 910 and 914 may not be distinct actions, and determining implant functionality may be applying transcutaneous current. For example, at 910, the external generator may apply or not apply therapeutic transcutaneous current based on whether the difference between the first impedance value and the second impedance value exceeds the first threshold value or the second threshold value.

The method above has been described above as being performed by the external generator. In some aspects, the method may be performed by a separate device.

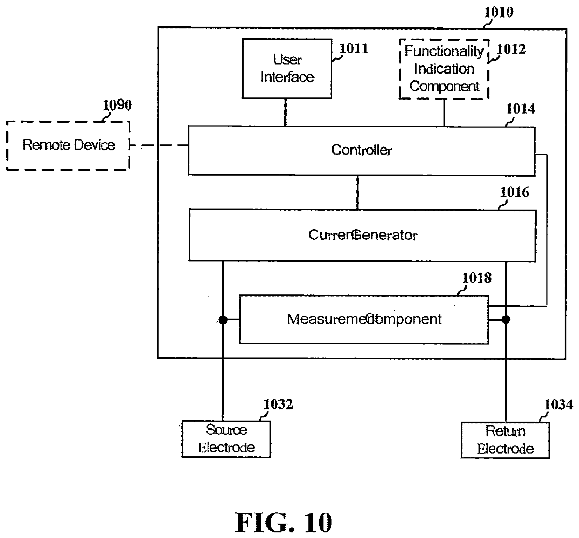

FIG. 10 is a block diagram 1000 illustrating an external generator 1010. For example, the external generator 1010 may be the external generator 110 of FIG. 1 or the external generator 510 of FIG. 5. The external generator 1010 includes a user interface 1011, a functionality indication component 1012, a controller 1014, a current generator 1016, a measurement component 1018, an active electrode 1032, and a return electrode 1034. The external generator 1010 may apply transcutaneous current to the skin of a subject, and an implantable device beneath the skin of the subject may present an electrical path which allows a portion of the applied transcutaneous current to path through the implantable device.

The current generator 1016 is coupled to the active electrode 1032 and the return electrode 1034. The current generator 1016 may include a constant current source or a constant voltage source. The current generator 1016 generates the current to be applied to the skin of the subject as transcutaneous current and applies the current between the active electrode 1032 and the return electrode 1034. For example, the current generator 1016 may generate bursts of alternating transcutaneous stimulation current and apply the bursts of alternating transcutaneous stimulation current between the active electrode 1032 and the return electrode 1034.

The measurement component 1018 is coupled to the active electrode 1032 and the return electrode 1034. The measurement component 1018 may measure the impedance between the active electrode 1032 and the return electrode 1034. In some aspects, the current generator 1016 may utilize a constant current source to generate the current applied between the active electrode 1032 and the return electrode 1034, and the measurement component 1018 may measure the voltage between the active electrode 1032 and the return electrode 1034 while the current is being applied to measure the impedance between the active electrode 1032 and the return electrode 1034. In some aspects, the current generator 1016 may utilize a constant voltage source to generate the current applied between the active electrode 1032 and the return electrode 1034, and the measurement component 1018 may measure the current passing between the active electrode 1032 and the return electrode 1034 while the current is being applied to measure the impedance between the active electrode 1032 and the return electrode 1034.

The controller 1014 is coupled to the current generator 1016. The controller 1014 may control when the current generator 1016 applies current between the active electrode 1032 and the return electrode 1034. The controller 1014 may control one or more attributes of the current applied by the current generator 1016. For example, the current generator 1016 may apply bursts of alternating current between the active electrode 1032 and the return electrode 1034, and the controller 1014 may set any of the amplitude, the carrier frequency, the burst duration, and the burst frequency of the current, or any combination thereof.

The controller 1014 is coupled to the measurement component 1018. The controller 1014 may control when the measurement component 1018 takes impedance measurements between the active electrode 1032 and the return electrode 1034.

In some aspects, the controller 1014 may be coupled to a functionality indication component 1012. The functionality indication component 1012 may be an element on the user interface 1011, may be an LED or other light, or may be a speaker. The controller 1014 may control the functionality indication component 1012 to output a functionality indication, as discussed further below.

In some aspects, the external generator 1010 may detect functionality in an implantable device without feedback from the implantable device based on measuring impedance between the active electrode 1032 and the return electrode 1034 for current applied in different directions. The current generator 1016 may apply a first current in a first direction between the active electrode 1032 and the return electrode 1034, for example, as described above with respect to 402 of FIG. 4. In some aspects, the current generator 1016 may apply therapeutic alternating transcutaneous current and the first current may be a portion of the therapeutic alternating transcutaneous current. In some aspects, the controller 1014 may specifically control the current generator 1016 to apply the first current. The measurement component 1018 may measure a first impedance based on the first current, for example, as described above with respect to 404 of FIG. 4. The current generator 1016 may apply a second current in a second direction between the active electrode 1032 and the return electrode 1034, for example, as described above with respect to 406 of FIG. 4. In some aspects, the current generator 1016 may apply therapeutic alternating transcutaneous current and the second current may be a portion of the therapeutic alternating transcutaneous current. In some aspects, the controller 1014 may specifically control the current generator 1016 to apply the second current. The measurement component 1018 may measure a second impedance based on the second current, for example, as described above with respect to 408 of FIG. 4. The controller 1014 may determine implant functionality for the implantable device based on the first impedance and the second impedance, for example, as described above with respect to 410 of FIG. 4.

In some aspects, the external generator 1010 may detect functionality in an implantable device without feedback from the implantable device based on measuring impedance between the active electrode 1032 and the return electrode 1034 for current applied with different amplitudes. The current generator 1016 may apply a first current having a first amplitude between the active electrode 1032 and the return electrode 1034, for example, as described above with respect to 802 of FIG. 8. The measurement component 1018 may measure a first impedance based on the first current, for example, as described above with respect to 804 of FIG. 8. The current generator 1016 may apply a second current having a second amplitude between the active electrode 1032 and the return electrode 1034, for example, as described above with respect to 806 of FIG. 8. The measurement component 1018 may measure a second impedance based on the second current, for example, as described above with respect to 808 of FIG. 8. The controller 1014 may determine implant functionality for the implantable device based on the first impedance and the second impedance, for example, as described above with respect to 810 of FIG. 8.

In some aspects, the external generator 1010 may detect functionality in an implantable device without feedback from the implantable device based on measuring impedance between the active electrode 1032 and the return electrode 1034 for current applied with different durations. The current generator 1016 may apply a first current having a first duration between the active electrode 1032 and the return electrode 1034, for example, as described above with respect to 902 of FIG. 9. The measurement component 1018 may measure a first impedance based on the first current, for example, as described above with respect to 904 of FIG. 9. The current generator 1016 may apply a second current having a second duration between the active electrode 1032 and the return electrode 1034, for example, as described above with respect to 906 of FIG. 9. The measurement component 1018 may measure a second impedance based on the second current, for example, as described above with respect to 908 of FIG. 9. The controller 1014 may determine implant functionality for the implantable device based on the first impedance and the second impedance, for example, as described above with respect to 910 of FIG. 9.

In some aspects, the controller 1016 may control the functionality indication component 1012 to output a functionality indication based on the determined implant functionality, for example as described above with respect to 412 of FIG. 4, 812 of FIG. 8, or 912 of FIG. 9. In some aspects, the controller 1016 may control the current generator 1016 to apply transcutaneous current based on the determined implant functionality, for example, as described above with respect to 414 of FIG. 4, 814 of FIG. 8, or 914 of FIG. 9.

In some aspects, the external generator 1010 may be coupled to a remote device 1090. For example, the controller 1014 may wirelessly communicate with the remote device 1090. The remote device 1090 may serve as a user interface for the system. For example, the remote device 1090 may be used to control the external generator, such as turning the application of current on or off, increasing or decreasing the amplitude of applied current, selecting a current program, and/or presenting the user with the functionality indication. In some aspects, the remote device 1090 may include communication components capable of communicating to a cloud-based system, for example, for storage or tracking of patient treatment data. In some aspects, the remote device 1090 may receive data from the external generator 1010 and may perform processing functions for the external generator 1010. For example, the external generator 1010 may transmit the first impedance value and the second impedance value to the remote device 1090, the remote device 1090 may determine the implant functionality based on the first impedance value and the second impedance value, and the remote device 1090 may transmit the implant functionality to the external generator 1010.

By way of example, one or more component of the block diagram discussed above (e.g., the controller 1014, or any portion of a component, or any combination of components may be implemented as a "processing system" that includes one or more processors. Examples of processors include microprocessors, microcontrollers, graphics processing units (GPUs), central processing units (CPUs), application processors, digital signal processors (DSPs), reduced instruction set computing (RISC) processors, systems on a chip (SoC), baseband processors, field programmable gate arrays (FPGAs), programmable logic devices (PLDs), state machines, gated logic, discrete hardware circuits, and other suitable hardware configured to perform the various functionality described throughout this disclosure. One or more processors in the processing system may be coupled to a computer readable medium storing software and may execute the software. Software shall be construed broadly to mean instructions, instruction sets, code, code segments, program code, programs, subprograms, software components, applications, software applications, software packages, routines, subroutines, objects, executables, threads of execution, procedures, functions, etc., whether referred to as software, firmware, middleware, microcode, hardware description language, or otherwise.

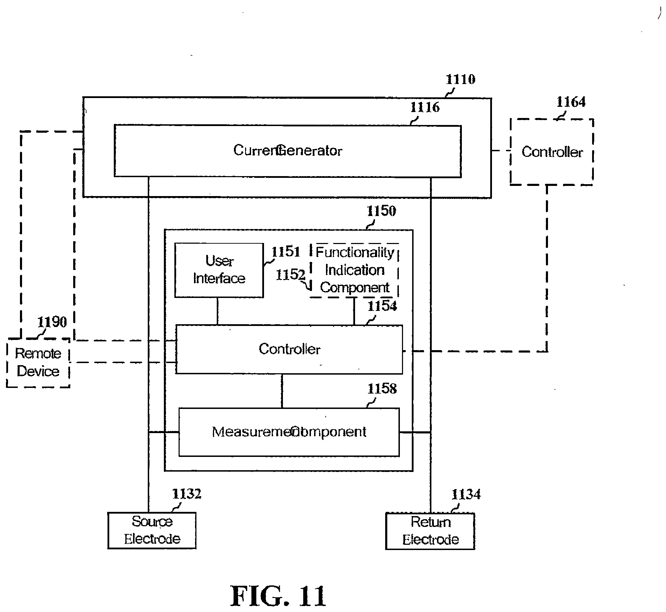

FIG. 11 is a block diagram illustrating an external generator 1110 and an implant monitor 1150. The external generator 1110 may be the external generator 110 of FIG. 1 or the external generator 510 of FIG. 5. The external generator 1110 includes a current generator 1116, an active electrode 1132, and a return electrode 1134. The implant monitor 1150 includes a user interface 1151, a functionality indication component 1152, a controller 1154, a current generator 1156, and a measurement component 1158. The external generator 1110 may apply transcutaneous current to the skin of a subject, and an implantable device beneath the skin of the subject may present an electrical path which allows a portion of the applied transcutaneous current to path through the implantable device. The implant monitor 1150 is a separate device that detects functionality in the implantable device based on the current applied by the external generator 1110.

The current generator 1116 of the external generator 1110 is coupled to the active electrode 1132 and the return electrode 1134. The current generator 1116 may include a constant current source or a constant voltage source. The current generator 1116 generates the current to be applied to the skin of the subject as transcutaneous current and applies the current between the active electrode 1132 and the return electrode 1134. For example, the current generator 1116 may generate bursts of alternating transcutaneous stimulation current and apply the bursts of alternating transcutaneous stimulation current between the active electrode 1132 and the return electrode 1134.

The measurement component 1158 of the implant monitor 1150 is coupled to the active electrode 1132 and the return electrode 1134 of the external generator 1110. The measurement component 1158 may measure the impedance between the active electrode 1132 and the return electrode 1134. In some aspects, the current generator 1116 may utilize a constant current source to generate the current applied between the active electrode 1132 and the return electrode 1134, and the measurement component 1158 may measure the voltage between the active electrode 1132 and the return electrode 1134 while the current is being applied to measure the impedance between the active electrode 1132 and the return electrode 1134. In some aspects, the current generator 1116 may utilize a constant voltage source to generate the current applied between the active electrode 1132 and the return electrode 1134, and the measurement component 1158 may measure the current passing between the active electrode 1132 and the return electrode 1134 while the current is being applied to measure the impedance between the active electrode 1132 and the return electrode 1134.

The controller 1154 is coupled to the measurement component 1158. The controller 1154 may control when the measurement component 1158 takes impedance measurements between the active electrode 1132 and the return electrode 1134.

In some aspects, the controller 1154 may be coupled to a functionality indication component 1152. The functionality indication component 1152 may be an element on the user interface 1151, may be an LED or other light, or may be a speaker. The controller 1154 may control the functionality indication component 1152 to output a functionality indication, as discussed further below.

The implant monitor 1150 may detect functionality in an implantable device without feedback from the implantable device based on measuring impedance between the active electrode 1132 and the return electrode 1134. In some aspects, the implant monitor 1150 may detect functionality in the implant based on measuring impedances for current applied in different directions. In some aspects, the implant monitor 1150 may detect functionality in the implant based on measuring impedances for current applied having different amplitudes. In some aspects, the implant monitor 1150 may detect functionality in the implant based on measuring impedances for current applied having different durations.

The current generator 1116 of the external generator 1110 may apply a first current between the active electrode 1132 and the return electrode 1134, for example, as described above with respect to 402 of FIG. 4, 802 of FIG. 8, or 902 of FIG. 9. In some aspects, the first current may be in a first direction, for example, as described above with respect to 402 of FIG. 4. In some aspects, the first current may have a first amplitude, as described above with respect to 802 of FIG. 8. In some aspects, the first current may have a first duration, as described above with respect to 902 of FIG. 9. In some aspects, the current generator 1116 may apply therapeutic alternating transcutaneous current and the first current may be a portion of the therapeutic alternating transcutaneous current. In some aspects, the controller 1154 may indicate to the external generator 1110 to apply the first current through a wired or wireless connection between the implant monitor 1150 and the external generator 1110. In some aspects, a separate controller 1164 may indicate to the external generator 1110 to apply the first current. For example, the separate controller 1164 may be a smartphone or other personal electronic device.

The measurement component 1158 may measure a first impedance based on the first current, for example, as described above with respect to 404 of FIG. 4, 804 of FIG. 8, or 904 of FIG. 9. In some aspects, the controller 1154 may monitor the current between the active electrode 1132 and the return electrode 1134 to detect the first current and may control the measurement component 1158 to measure the first impedance when the first current is detected. In some aspects, the controller 1154 may indicate to the external generator 1110 to apply the first current and may control the measurement component 1158 to measure the first impedance based on having indicated to the external generator 1110 to apply the first current. In some aspects, the external generator 1110 may transmit an indication to the implant monitor 1150 that the external generator 1110 will apply the first current, and the controller 1154 may control the measurement component 1158 to measure the first impedance based on having received the indication. In some aspects, the separate controller 1164 may indicate to the external generator 1110 to apply the first current and may indicate to the implant monitor 1150 to measure the first impedance, and the controller 1154 may control the measurement component 1158 to measure the first impedance based on having received the indication.

The current generator 1116 may apply a second current between the active electrode 1132 and the return electrode 1134, for example, as described above with respect to 406 of FIG. 4, 806 of FIG. 8, or 906 of FIG. 9. In some aspects, the second current may be in a second direction, for example, as described above with respect to 406 of FIG. 4. In some aspects, the second current may have a second amplitude, as described above with respect to 806 of FIG. 8. In some aspects, the second current may have a second duration, as described above with respect to 906 of FIG. 9. In some aspects, the current generator 1116 may apply therapeutic alternating transcutaneous current and the second current may be a portion of the therapeutic alternating transcutaneous current. In some aspects, the controller 1154 may indicate to the external generator 1110 to apply the second current through a wired or wireless connection between the implant monitor 1150 and the external generator 1110. In some aspects, a separate controller 1164 may indicate to the external generator 1110 to apply the second current.

The measurement component 1158 may measure a second impedance based on the second current, for example, as described above with respect to 408 of FIG. 4, 808 of FIG. 8, or 908 of FIG. 9. In some aspects, the controller 1154 may monitor the current between the active electrode 1132 and the return electrode 1134 to detect the second current and may control the measurement component 1158 to measure the second impedance when the second current is detected. In some aspects, the controller 1154 may indicate to the external generator 1110 to apply the second current and may control the measurement component 1158 to measure the second impedance based on having indicated to the external generator 1110 to apply the second current. In some aspects, the external generator 1110 may transmit an indication to the implant monitor 1150 that the external generator 1110 will apply the second current, and the controller 1154 may control the measurement component 1158 to measure the second impedance based on having received the indication. In some aspects, the separate controller 1164 may indicate to the external generator 1110 to apply the second current and may indicate to the implant monitor 1150 to measure the second impedance, and the controller 1154 may control the measurement component 1158 to measure the second impedance based on having received the indication.

The controller 1154 may determine implant functionality for the implantable device based on the first impedance and the second impedance, for example, as described above with respect to 410 of FIG. 4, 810 of FIG. 8, or 910 of FIG. 9.

In some aspects, the controller 1154 may control the functionality indication component 1152 to output a functionality indication based on the determined implant functionality, for example as described above with respect to 412 of FIG. 4, 812 of FIG. 8, or 912 of FIG. 9. In some aspects, the controller 1154 transmit an indication to the external generator 1110 to apply transcutaneous current based on the determined implant functionality, for example, as described above with respect to 414 of FIG. 4, 814 of FIG. 8, or 914 of FIG. 9.

In some aspects, the external generator 1110 and/or the implant monitor 1150 may be coupled to a remote device 1190. For example, the external generator 1110 or the controller 1154 may wirelessly communicate with the remote device 1190. The remote device 1190 may serve as a user interface for the system. For example, the remote device 1190 may be used to control the external generator 1110, such as turning the application of current on or off, increasing or decreasing the amplitude of applied current, selecting a current program, and/or presenting the user with the functionality indication. In some aspects, the remote device 1190 may include communication components capable of communicating to a cloud-based system, for example, for storage or tracking of patient treatment data. In some aspects, the remote device 1190 may receive data from the implant monitor 1150 and may perform processing functions for the implant monitor 1150. For example, the implant monitor 1150 may transmit the first impedance value and the second impedance value to the remote device 1190, the remote device 1190 may determine the implant functionality based on the first impedance value and the second impedance value, and the remote device 1190 may transmit the implant functionality to the implant monitor 1150.

By way of example, one or more component of the block diagram discussed above (e.g., the controller 1114), or any portion of a component, or any combination of components may be implemented as a "processing system" that includes one or more processors. Examples of processors include microprocessors, microcontrollers, graphics processing units (GPUs), central processing units (CPUs), application processors, digital signal processors (DSPs), reduced instruction set computing (RISC) processors, systems on a chip (SoC), baseband processors, field programmable gate arrays (FPGAs), programmable logic devices (PLDs), state machines, gated logic, discrete hardware circuits, and other suitable hardware configured to perform the various functionality described throughout this disclosure. One or more processors in the processing system may be coupled to a computer readable medium storing software and may execute the software. Software shall be construed broadly to mean instructions, instruction sets, code, code segments, program code, programs, subprograms, software components, applications, software applications, software packages, routines, subroutines, objects, executables, threads of execution, procedures, functions, etc., whether referred to as software, firmware, middleware, microcode, hardware description language, or otherwise.

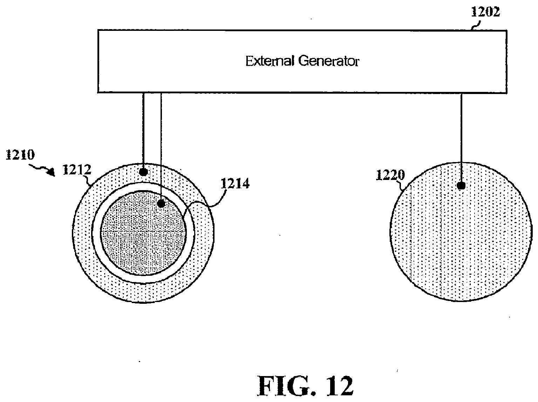

FIG. 12 is a diagram of an external generator 1202 capable of applying current using different electrode surface areas. The external generator 1202 includes a source electrode 1210 and a return electrode 1220.

The external generator 1202 includes a segmented source electrode 1210. The source electrode 1210 includes a first segment 1212 and a second segment 1214. The first segment 1212 and the second segment 1214 may be electrically isolated from one another. The external generator 1202 may electrically coupled to the first segment 1212 and may be separately electrically connected to the second segment 1214. In some aspects, a segmented electrode may include two segments having a shape of concentric circles. In some aspects, a segmented electrode may have more than two segments, and in some aspects the segments of the segmented electrode may have other shapes.

When the first segment 1212 of the source electrode 1210, the second segment 1214 of the source electrode 1210, and the return electrode 1220 are in contact with the skin of a subject, the external generator may utilize different combinations of electrodes coupled to the terminals of the current generator to apply transcutaneous current using different electrode surface areas.

In some aspects, to apply transcutaneous current using a first electrode surface area, the external generator 1202 may connect both the first segment 1212 and the second segment 1214 of the source electrode to one terminal of the current generator and may connect the return electrode 1220 to the other terminal of the current generator. To apply transcutaneous current using a second electrode surface area, the external generator 1202 may connect one of the first segment 1212 or the second segment 1214 of the source electrode 1210 to one terminal of the current generator and may connect the return electrode 1220 to the other terminal of the current generator.

In some aspects, to apply transcutaneous current using a first electrode surface area, the external generator 1202 may connect the first segment 1212 of the source electrode 1210 to one terminal of the current generator and may connect the return electrode 1220 to the other terminal of the current generator. To apply transcutaneous current using a second electrode surface area, the external generator 1202 may connect the second segment 1214 of the source electrode 1210 to one terminal of the current generator and may connect the return electrode 1220 to the other terminal of the current generator.

Referring to FIGS. 3 and 7 above, the electrode-skin impedance represented by the first resistors Rs may be inversely proportional to the contact area between the electrode and the skin. For example, if the electrode surface area is increased by a factor of two, the electrode-skin impedance may decrease by the same factor of two. By utilizing different electrode surface areas with known surface area ratios, the external generator may measure the impedance between the electrodes of the external generator with different values of the electrode-skin impedance.

As will be discussed further below, the external generator 1202 may utilize the ratio of the surface areas to determine a pick up ratio of an implantable device or to determine the current passing through an implantable device. In some aspects, the ratio of the surface areas may be known (e.g., may have been measured) and may be preconfigured for the external generator. In some aspects, the external generator 1202 may determine the ratio of the surface areas. The electrode-skin impedance may have a capacitive component, and the capacitive component may be proportional to the surface area. The external generator 1202 may determine the capacitive component of the electrode-skin impedance for the first electrode surface area, may determine the capacitive component of the electrode-skin impedance for the second electrode surface area, and may determine the ratio of surface areas to be the ratio of the capacitive components. In some aspects, the external generator 1202 may apply sine voltage or sine current between the source electrode 1210 and the return electrode 1220 using a particular electrode surface area, may measure the voltage applied and the corresponding current, and may calculate the capacitive component for the particular electrode surface area based on the measured voltage and current or based on the amplitude and phase difference between the voltage and the current. In some aspects, the external generator 1202 may apply a pulse of constant current between the source electrode 1210 and the return electrode 1220 using a particular electrode surface area, may measure the voltage corresponding to the applied current, and may calculate the capacitive component for the particular electrode surface area based on the applied current and measured voltage.

As discussed above, the terms "source electrode" and "return electrode" are used herein for clarity to differentiate where the electrode is placed with respect to the implantable device and to clarify the meaning of "positive" and "negative" current. The external generator 1202 of FIG. 12 is described as having a segmented source electrode 1210 and a unitary return electrode 1220. In some aspects, an external generator may include a unitary source electrode (e.g., the unitary electrode may be placed above the implantation site of a receiving electrode of the implantable device) and a segmented return electrode (e.g., the return electrode may be placed at a different location, such as above or near the implantation site of the output electrodes of the implantable device) and may be able to apply current utilizing different electrode surface areas as described above. In some aspects, an external generator may include both a segmented source electrode and a segmented return electrode.

In some alternative aspects, an external generator may include two source electrodes attached in close proximity to each other on the skin of the subject, and/or two return electrodes attached in close proximity to each other on the skin of the subject, and may utilize each electrode as a different segment as described above.

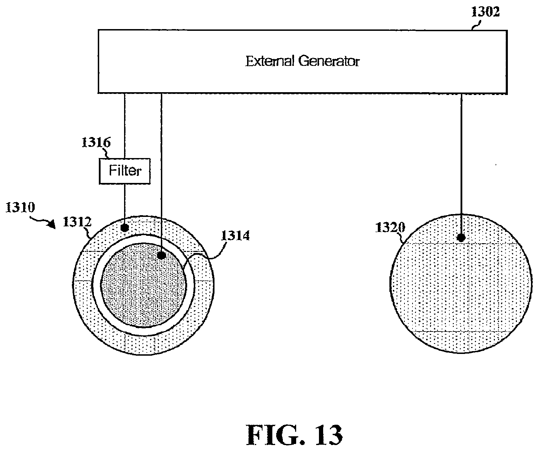

FIG. 13 is a diagram of an external generator 1302 utilizing a frequency selected segmented electrode. The external generator 1302 includes an active electrode 1310 and a return electrode 1320.

The external generator 1302 includes a segmented active electrode 1310. The active electrode 1310 includes a first segment 1312 and a second segment 1314. The first segment 1312 and the second segment 1314 may be electrically isolated from one another. The first segment 1312 is coupled to the external generator 1302 through a filter 1316. The filter 1316 may be a band pass filter, a band stop filter, a low pass filter, a high pass filter, or a notch filter. The second segment 1314 may be coupled directly to the external generator 1302. The return electrode 1320 may be coupled directly to the external generator 1302.

The external generator 1302 may apply transcutaneous current having different carrier frequencies to utilize different electrode surface areas. The external generator 1302 may apply transcutaneous current between the active electrode 1310 and the return electrode 1320 having a first carrier frequency which passes the filter 1316 to utilize an electrode surface area that includes both the first segment 1312 and the second segment of the active electrode 1310. The external generator 1302 may apply transcutaneous current between the active electrode 1310 and the return electrode 1320 having a carrier frequency which does not pass the filter 1316 to utilize an electrode surface area that includes the second segment 1314 of the active electrode 1310, but does not include the first segment 1312 of the active electrode 1310.

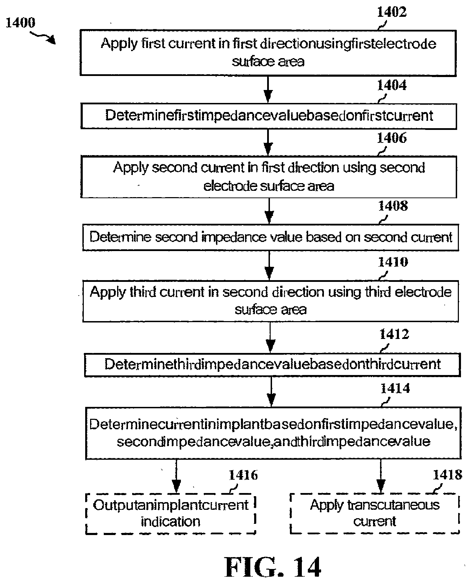

FIG. 14 is a flowchart 1400 illustrating a method of determining the current passing through an implantable device. In some aspects, the method may be performed by an external generator (e.g., the external generator 110 of FIG. 1). The external generator may be able to apply current using different electrode surface areas as described above with respect to the external generator 1202 of FIG. 12.

An implantable device (e.g., the implantable device 130 of FIG. 1) may be implanted beneath the skin of a subject to present a path for current from the external generator. The implantable device may include a component with a different forward and backward impedance in the path of the current (e.g., a diode).

At 1402, the external generator may apply a first current in a first direction to the skin of the subject. The first current may be in the direction for which the component with a different forward and backward impedance has a high impedance. For example, the component may be a diode and the first current may be in the direction that the diode does not conduct. The external generator may apply the first current utilizing a first electrode surface area.

At 1404, the external generator determines a first impedance value based on the first current. The first impedance value may be or may correspond to the impedance between the source electrode and the return electrode when the external generator is applying the first current in the first direction. In some aspects, the external generator may measure the impedance between the source electrode and the return electrode while the first current is being applied to determine the first impedance value. In some aspects, the external generator may measure the voltage or the current between the source electrode and the return electrode while the first current is being applied to determine the first impedance value. In some aspects, the external generator may receive the first impedance value from another device which measured the impedance, voltage, or current between the source electrode an the return electrode when the first current was being applied.

At 1406, the external generator may apply a second current in the first direction to the skin of the subject. The external generator may apply the second current utilizing a second electrode surface area which is different than the first electrode surface area utilized to apply the first current. For example, the external generator may apply the first current utilizing a first segment of a segmented electrode and may apply the second current utilizing a second segment of the segmented electrode, or the external generator may apply the first current utilizing a first segment of a segmented electrode and may apply the second current utilizing both the fist segment and the second segment of the segmented electrode.

At 1408, the external generator determines a second impedance value based on the second current. The second impedance value may be the impedance between the source electrode and the return electrode when the external generator is applying the second current in the first direction. In some aspects, the external generator may measure the impedance between the source electrode and the return electrode while the second current is being applied to determine the second impedance value. In some aspects, the external generator may measure the voltage or the current between the source electrode and the return electrode while the second current is being applied to determine the second impedance value. In some aspects, the external generator may receive the second impedance value from another device which measured the impedance, voltage, or current between the source electrode an the return electrode when the second current was being applied.

At 1410, the external generator may apply a third current in a second direction to the skin of the subject. The second direction may be different than the first direction. The first direction may be the direction for which the component with a different forward and backward impedance has a high impedance, and the second direction may be the direction for which the component with a different forward and backward impedance has a low impedance. For example, the component may be a diode, the first current may be in the direction that the diode does not conduct, and the second direction may be the direction that the diode does conduct. The external generator may apply the first current utilizing a third electrode surface area. In some aspects, the third surface area may be the same as the first surface area. In some aspects, the third surface area may be the same as the second surface area. In some aspects, the third surface area may be different than both the first surface area and the second surface area.

At 1412, the external generator determines a third impedance value based on the third current. The third impedance value may be or may correspond to the impedance between the source electrode and the return electrode when the external generator is applying the third current in the second direction. In some aspects, the external generator may measure the impedance between the source electrode and the return electrode while the third current is being applied to determine the third impedance value. In some aspects, the external generator may measure the voltage or the current between the source electrode and the return electrode while the third current is being applied to determine the third impedance value. In some aspects, the external generator may receive the third impedance value from another device which measured the impedance, voltage, or current between the source electrode an the return electrode when the third current was being applied.

At 1414, the external generator determines how much of the transcutaneous current applied by the external generator passes through the implantable device based on the first impedance value determined at 1404, the second impedance value determined at 1408, and the third impedance value determined at 1412. For example, the external generator may determine an implant current corresponding to the amplitude of current passing through the implantable device and/or may determine a pick up ratio for the implantable device.

The external generator may utilize the first impedance value and the second impedance value to determine the tissue path impedance and the skin-electrode impedances of the first impedance value, the second impedance value, and the third impedance value. Both the first impedance value and the second impedance value are determined when the component with a different forward and backward impedance is not conducting. Accordingly, the determined impedance equals the tissue path impedance plus the skin-electrode impedance. The skin-electrode impedances are both inversely proportional to the electrode surface area used to obtain the determined impedance, so the known ratio of the first electrode surface area results in a known ratio of the skin-electrode impedance component of the first impedance value (e.g., the skin-electrode impedance component corresponding to the first electrode surface area) to the skin-electrode impedance component of the second impedance value (e.g., the skin-electrode impedance value corresponding to the second electrode surface area). Utilizing these relationships, the external generator can determine the tissue path impedance and the skin-electrode impedances.

For example, the first electrode surface area may be half the second electrode surface area. The tissue path impedance may be referred to as Rt, the skin-electrode impedance when utilizing the first surface area may be referred to as Rs, the first impedance value may be referred to as Z1, and the second impedance value may be referred to as Z2. The external generator may utilize the formulas Z1 = Rt + Rs and Z2 = Rt + (2*Rs) to solve for Rt and Rs.

The external generator may utilize the tissue path impedance, the skin-electrode impedances, and the third impedance value determines how much of the transcutaneous current applied by the external generator passes through the implantable device. The third impedance value is determined when the component with a different forward and backward impedance is conducting. Accordingly, the determined impedance is based on the tissue path impedance, the skin-electrode impedance, the implant path impedance, and the implant impedance. The external generator determined the tissue path impedance and the skin-electrode impedance based on the first impedance value and the second impedance value. The external generator may therefore determine the combined implant path impedance and implant impedance.

For example, the third impedance corresponding to the third impedance value may be referred to as Z3. The combined implant path impedance and implant impedance may be referred to as Ri. The third electrode surface area may be the same as the first electrode surface area, so the skin-electrode impedance component for the third impedance value may be Rs. The external generator may utilize the formula Ri = ((Z1 * Rt) - (Rs*Rt)) / (Rs + Rt - Z1) to determine the combined implant path impedance and implant impedance Ri.

The external generator may utilize the known tissue path impedance, the skin-electrode impedance, and the combined implant path impedance and implant impedance to determine the pick-up ratio of the implantable device. The pick-up ratio is defined as the ratio between the current flowing via the implantable device to the total transcutaneous current delivered by the external stimulator. For example, the external generator may utilize the formula Rt / (Ri + Rt) to determine the pick-up ratio of the implantable device. The external generator may utilize the pick-up ratio of the implantable device and the transcutaneous current applied by the external generator to determine the current passing through the implantable device.

In some aspects, the external generator may determine how much of the transcutaneous current applied by the external generator passes through the implantable device based on the first impedance value, the second impedance value, and the third impedance value by transmitting the first impedance value, the second impedance value, and the third impedance value to an external device (e.g., a remote control or a smart phone) and receiving the implant current value or pick up ratio from the external device.

In some aspects, at 1416, the external generator may output an implant current indication based on the current in the implantable device determined at 1414. The external generator may utilize an implant current indication component to output the implant current indication. For example, the implant current indication component may be an indicator light on the external generator and outputting the implant current indication may be lighting or not lighting the indicator light based on the implant current or the pick up ratio exceeding a threshold value or falling within a specified range. The implant current indication component may be a display of a user interface and outputting the implant current indication may be displaying the implant current or the implant pick up ratio on the display. The implant current indication component may be a speaker and outputting the implant current indication may be utilizing the speaker to make a sound based on the implant current or the implant pick up ratio exceeding a threshold value or falling within a specified range. In some aspects, the external generator may output the implant current indication by transmitting the implant current indication to an external device such as a remote control or a smart phone in communication with the external generator.

In some aspects, at 1418, the external generator may apply transcutaneous current based on the implant current determined at 1414. For example, the external generator may compare the implant current determined at 1414 to a target current value, and may apply transcutaneous current having an amplitude based on the comparison (e.g., may increase the amplitude of applied transcutaneous current from previous values if the implant current is lower than the target current value or may decrease the amplitude of applied transcutaneous current from previous values if the implant current is higher than the target current value).

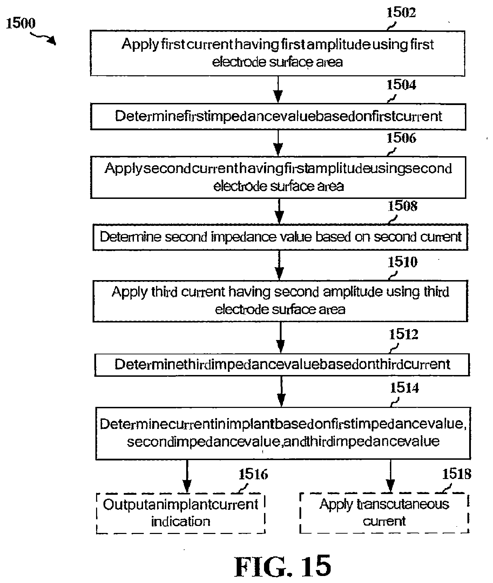

FIG. 15 is a flowchart 1500 illustrating a method of determining the current passing through an implantable device. In some aspects, the method may be performed by an external generator (e.g., the external generator 110 of FIG. 1 or the external generator 510 of FIG. 5). The external generator may be able to apply current using different electrode surface areas as described above with respect to the external generator 1202 of FIG. 12.

An implantable device (e.g., the implantable device 130 of FIG. 1 or the implantable device 530 of FIG. 5) is implanted beneath the skin of a subject to present a path for current from the external generator. In some aspects, the implantable device may include a single channel, and the channel may include a diode rectifying current passing through the implantable device. In some aspects, the implantable device may include multiple channels, and each channel may include a diode rectifying current passing through that channel.

At 1502, the external generator may apply a first current having a first amplitude to the skin of the subject. The first current may be alternating transcutaneous current. The first amplitude of the first current is low enough that it does not result in the diode or diodes of the implantable device being forward biased during application of the first current. In some aspects, the first amplitude may be between .1 mA and 10 mA. In some aspects, the first amplitude may be between .9 mA and 1.1 mA. In some aspects, the first amplitude may be 1 mA. The external generator may apply the first current utilizing a first electrode surface area.

At 1504, the external generator determines a first impedance value based on the first current. The first impedance value may be or may correspond to the impedance between the source electrode and the return electrode when the external generator is applying the first current in the first direction. In some aspects, the external generator may measure the impedance between the source electrode and the return electrode while the first current is being applied to determine the first impedance value. In some aspects, the external generator may measure the voltage or the current between the source electrode and the return electrode while the first current is being applied to determine the first impedance value. In some aspects, the external generator may receive the first impedance value from another device which measured the impedance, voltage, or current between the source electrode an the return electrode when the first current was being applied.

At 1506, the external generator may apply a second current having the first amplitude to the skin of the subject. The external generator may apply the second current utilizing a second electrode surface area which is different than the first electrode surface area utilized to apply the first current. For example, the external generator may apply the first current utilizing a first segment of a segmented electrode and may apply the second current utilizing a second segment of the segmented electrode, or the external generator may apply the first current utilizing a first segment of a segmented electrode and may apply the second current utilizing both the fist segment and the second segment of the segmented electrode.

At 1508, the external generator determines a second impedance value based on the second current. The second impedance value may be or may correspond to the impedance between the source electrode and the return electrode when the external generator is applying the second current in the first direction. In some aspects, the external generator may measure the impedance between the source electrode and the return electrode while the second current is being applied to determine the second impedance value. In some aspects, the external generator may measure the voltage or the current between the source electrode and the return electrode while the second current is being applied to determine the second impedance value. In some aspects, the external generator may receive the second impedance value from another device which measured the impedance, voltage, or current between the source electrode and the return electrode when the second current was being applied.

At 1510, the external generator may apply a third current having a second amplitude to the skin of the subject. The second current may be alternating transcutaneous current. The second amplitude of the second current may be high enough that it forward biases the diode or diodes in the implantable device. In some aspects, the second amplitude may be between 5 mA and 500 mA. In some aspects, the second amplitude may be between 40 mA and 60 mA. In some aspects, the second amplitude may be 50 mA. The external generator may apply the third current utilizing a third electrode surface area. In some aspects, the third surface area may be the same as the first surface area. In some aspects, the third surface area may be the same as the second surface area. In some aspects, the third surface area may be different than both the first surface area and the second surface area.

At 1512, the external generator determines a third impedance value based on the third current. The third impedance value may be or may correspond to the impedance between the source electrode and the return electrode when the external generator is applying the third current. In some aspects, the external generator may measure the impedance between the source electrode and the return electrode while the third current is being applied to determine the third impedance value. In some aspects, the external generator may measure the voltage or the current between the source electrode and the return electrode while the third current is being applied to determine the third impedance value. In some aspects, the external generator may receive the third impedance value from another device which measured the impedance, voltage, or current between the source electrode and the return electrode when the third current was being applied.

At 1514, the external generator determines how much of the transcutaneous current applied by the external generator passes through the implantable device based on the first impedance value determined at 1504, the second impedance value determined at 1508, and the third impedance value determined at 1512. The external generator may determine how much of the transcutaneous current passes through the implantable device as described above with respect to 1414 of FIG. 14.

In some aspects, the external generator may determine how much of the transcutaneous current applied by the external generator passes through the implantable device based on the first impedance value, the second impedance value, and the third impedance value by transmitting the first impedance value, the second impedance value, and the third impedance value to an external device (e.g., a remote control or a smart phone) and receiving the implant current value or pick up ratio from the external device.

In some aspects, at 1516, the external generator may output an implant current indication based on the current in the implantable device determined at 1514. The external generator may utilize an implant current indication component to output the implant current indication. For example, the implant current indication component may be an indicator light on the external generator and outputting the implant current indication may be lighting or not lighting the indicator light based on the implant current or the pick up ratio exceeding a threshold value or falling within a specified range. The implant current indication component may be a display of a user interface and outputting the implant current indication may be displaying the implant current or the implant pick up ratio on the display. The implant current indication component may be a speaker and outputting the implant current indication may be utilizing the speaker to make a sound based on the implant current or the implant pick up ratio exceeding a threshold value or falling within a specified range. In some aspects, the external generator may output the implant current indication by transmitting the implant current indication to an external device such as a remote control or a smart phone in communication with the external generator.

In some aspects, at 1518, the external generator may apply transcutaneous current based on the implant current determined at 1514. For example, the external generator may compare the implant current determined at 1514 to a target current value, and may apply transcutaneous current having an amplitude based on the comparison (e.g., may increase the amplitude of applied transcutaneous current from previous values if the implant current is lower than the target current value or may decrease the amplitude of applied transcutaneous current from previous values if the implant current is higher than the target current value).

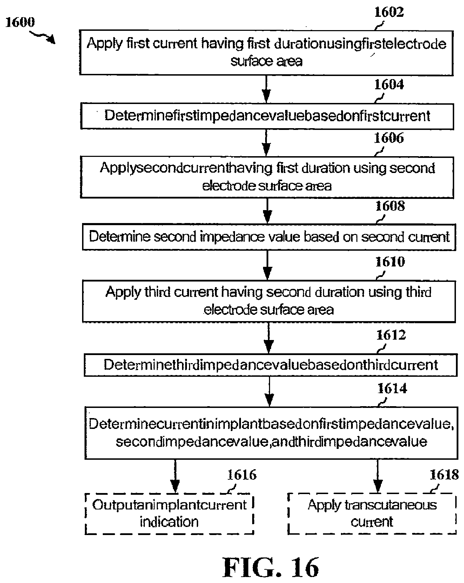

FIG. 16 is a flowchart 1600 illustrating a method of determining the current passing through an implantable device. In some aspects, the method may be performed by an external generator (e.g., the external generator 110 of FIG. 1 or the external generator 510 of FIG. 5). The external generator may be able to apply current using different electrode surface areas as described above with respect to the external generator 1202 of FIG. 12.

An implantable device (e.g., the implantable device 130 of FIG. 1 or the implantable device 530 of FIG. 5) is implanted beneath the skin of a subject to present a path for current from the external generator. In some aspects, the implantable device may include a single channel, and the channel may include a capacitor. In some aspects, the implantable device may include multiple channels, and each channel may include a capacitor.

At 1602, the external generator may apply a first current having a first duration to the skin of the subject. The first current may be alternating transcutaneous current. In some aspects, the first duration of the second current is long enough that capacitor or capacitors in the implantable device may reach high charge over the first duration. In some aspects, the first duration of the first current is long enough that the second current may charge the capacitor or capacitors of the implantable device to have effective impedances of their impedances at full charge. In some aspects, the first duration may be greater than [one second].

At 1604, the external generator determines a first impedance value based on the first current. The first impedance value may be or may correspond to the impedance between the source electrode and the return electrode when the external generator is applying the first current in the first direction. In some aspects, the external generator may measure the impedance between the source electrode and the return electrode while the first current is being applied to determine the first impedance value. In some aspects, the external generator may measure the voltage or the current between the source electrode and the return electrode while the first current is being applied to determine the first impedance value. In some aspects, the external generator may receive the first impedance value from another device which measured the impedance, voltage, or current between the source electrode an the return electrode when the first current was being applied.

At 1606, the external generator may apply a second current having the first duration to the skin of the subject. The external generator may apply the second current utilizing a second electrode surface area which is different than the first electrode surface area utilized to apply the first current. For example, the external generator may apply the first current utilizing a first segment of a segmented electrode and may apply the second current utilizing a second segment of the segmented electrode, or the external generator may apply the first current utilizing a first segment of a segmented electrode and may apply the second current utilizing both the first segment and the second segment of the segmented electrode.

At 1608, the external generator determines a second impedance value based on the second current. The second impedance value may be or may correspond to the impedance between the source electrode and the return electrode when the external generator is applying the second current in the first direction. In some aspects, the external generator may measure the impedance between the source electrode and the return electrode while the second current is being applied to determine the second impedance value. In some aspects, the external generator may measure the voltage or the current between the source electrode and the return electrode while the second current is being applied to determine the second impedance value. In some aspects, the external generator may receive the second impedance value from another device which measured the impedance, voltage, or current between the source electrode and the return electrode when the second current was being applied.

At 1610, the external generator may apply a third current having a second duration to the skin of the subject. The second current may be alternating transcutaneous current. In some aspects, the second duration of the third current is short enough that the third current may build little or no charge on the capacitor or capacitors of the implantable device. In some aspects, the second duration of the third current is short enough that the third current does cause the capacitor or capacitors to have high effective impedances. In some aspects, applying the third current over the second duration may be applying a single cycle of alternating current.

At 1612, the external generator determines a third impedance value based on the third current. The third impedance value may be or may correspond to the impedance between the source electrode and the return electrode when the external generator is applying the third current. In some aspects, the external generator may measure the impedance between the source electrode and the return electrode while the third current is being applied to determine the third impedance value. In some aspects, the external generator may measure the voltage or the current between the source electrode and the return electrode while the third current is being applied to determine the third impedance value. In some aspects, the external generator may receive the third impedance value from another device which measured the impedance, voltage, or current between the source electrode and the return electrode when the third current was being applied.

At 1614, the external generator determines how much of the transcutaneous current applied by the external generator passes through the implantable device based on the first impedance value determined at 1604, the second impedance value determined at 1608, and the third impedance value determined at 1612. The external generator may determine how much of the transcutaneous current passes through the implantable device as described above with respect to 1414 of FIG. 14.

In some aspects, the external generator may determine how much of the transcutaneous current applied by the external generator passes through the implantable device based on the first impedance value, the second impedance value, and the third impedance value by transmitting the first impedance value, the second impedance value, and the third impedance value to an external device (e.g., a remote control or a smart phone) and receiving the implant current value or pick up ratio from the external device.

In some aspects, at 1616, the external generator may output an implant current indication based on the current in the implantable device determined at 1614. The external generator may utilize an implant current indication component to output the implant current indication. For example, the implant current indication component may be an indicator light on the external generator and outputting the implant current indication may be lighting or not lighting the indicator light based on the implant current or the pick up ratio exceeding a threshold value or falling within a specified range. The implant current indication component may be a display of a user interface and outputting the implant current indication may be displaying the implant current or the implant pick up ratio on the display. The implant current indication component may be a speaker and outputting the implant current indication may be utilizing the speaker to make a sound based on the implant current or the implant pick up ratio exceeding a threshold value or falling within a specified range. In some aspects, the external generator may output the implant current indication by transmitting the implant current indication to an external device such as a remote control or a smart phone in communication with the external generator.

In some aspects, at 1618, the external generator may apply transcutaneous current based on the implant current determined at 1614. For example, the external generator may compare the implant current determined at 1614 to a target current value, and may apply transcutaneous current having an amplitude based on the comparison (e.g., may increase the amplitude of applied transcutaneous current from previous values if the implant current is lower than the target current value or may decrease the amplitude of applied transcutaneous current from previous values if the implant current is higher than the target current value).

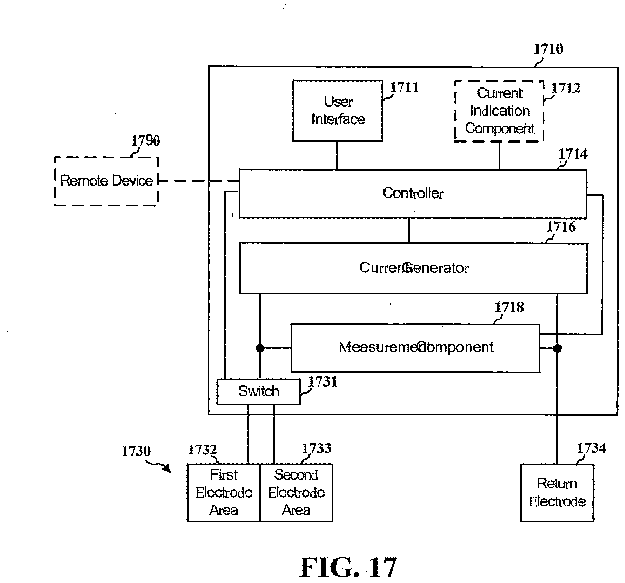

FIG. 17 is a block diagram illustrating an external generator 1710. For example, the external generator 1702 may be the external generator 110 of FIG. 1 or the external generator 510 of FIG. 5. The external generator 1710 includes a user interface 1711, a current indication component 1712, a controller 1714, a current generator 1716, a measurement component 1718, an active electrode 1730, a return electrode 1734, and a switching component 1731. The external generator 1710 may apply transcutaneous current to the skin of a subject, and an implantable device beneath the skin of the subject may present an electrical path which allows a portion of the applied transcutaneous current to path through the implantable device.

The current generator 1716 is coupled to the active electrode 1730 and the return electrode 1734. The current generator 1716 may include a constant current source or a constant voltage source. The current generator 1716 generates the current to be applied to the skin of the subject as transcutaneous current and applies the current between the active electrode 1730 and the return electrode 1734. For example, the current generator 1716 may generate bursts of alternating transcutaneous stimulation current and apply the bursts of alternating transcutaneous stimulation current between the active electrode 1730 and the return electrode 1734.

The measurement component 1718 is coupled to the active electrode 1730 and the return electrode 1734. The measurement component 1718 may measure the impedance between the active electrode 1730 and the return electrode 1734. In some aspects, the current generator 1716 may utilize a constant current source to generate the current applied between the active electrode 1730 and the return electrode 1734, and the measurement component 1718 may measure the voltage between the active electrode 1730 and the return electrode 1734 while the current is being applied to measure the impedance between the active electrode 1730 and the return electrode 1734. In some aspects, the current generator 1716 may utilize a constant voltage source to generate the current applied between the active electrode 1730 and the return electrode 1734, and the measurement component 1718 may measure the current passing between the active electrode 1730 and the return electrode 1734 while the current is being applied to measure the impedance between the active electrode 1730 and the return electrode 1734.

The controller 1714 is coupled to the current generator 1716. The controller 1714 may control when the current generator 1716 applies current between the active electrode 1730 and the return electrode 1734. The controller 1714 may control one or more attributes of the current applied by the current generator 1716. For example, the current generator 1716 may apply bursts of alternating current between the active electrode 1730 and the return electrode 1734, and the controller 1714 may set any of the amplitude, the carrier frequency, the burst duration, and the burst frequency of the current, or any combination thereof.

The controller 1714 is coupled to the measurement component 1718. The controller 1714 may control when the measurement component 1718 takes impedance measurements between the active electrode 1730 and the return electrode 1734.

The external generator 1710 is capable of applying transcutaneous current between the active electrode 1730 and the return electrode 1734 utilizing different electrode surface areas. The active electrode 1730 includes a first electrode area 1732 and a second electrode area 1733. The first electrode area 1732 and the second electrode area 1733 may be electrically separate. The switching component 1731 is coupled between the first electrode area 1732 and the current generator 1716 and is coupled between the second electrode area 1733 and the current generator 1716. The switching component 1731 may control whether the first electrode area 1732 and the second electrode area 1733 are electrically coupled to the current generator 1716. For example, the switching component 1731 may include one or more mechanical switch or one or more transistor. The controller 1714 is coupled to the switching component 1731 and may control which electrode areas of the active electrode 1730 the switching component 1731 electrically couples to the current generator 1716.

In some aspects, the controller 1714 may be coupled to a current indication component 1712. The current indication component 1712 may be an element on the user interface 1011, may be an LED or other light, or may be a speaker. The controller 1714 may control the current indication component 1712 to output a current indication, as discussed further below.

The external generator 1710 may determine the pick up ratio of the implantable device or the amount of current passing through the implantable device without feedback from the implantable device. In some aspects, the external generator 1710 may determine the pick up ratio of the implantable device or the amount of current passing through the implantable device based on measuring impedance between the active electrode 1730 and the return electrode 1734 for current applied in different directions with different electrode surface areas. The current generator 1716 may apply a first current between the active electrode 1730 and the return electrode 1734 in a first direction using a first electrode surface area, for example, as described above with respect to 1402 of FIG. 14. The controller 1714 may control the switching component 1731 to couple the electrode surface area or electrode surface areas corresponding to the first electrode surface area to the current generator 1716. The measurement component 1718 may measure a first impedance based on the first current, as described above with respect to 1404 of FIG. 14. The current generator 1716 may apply a second current between the active electrode 1730 and the return electrode 1734 in the first direction using a second electrode surface area, for example, as described above with respect to 1406 of FIG. 14. The controller 1714 may control the switching component 1731 to couple the electrode surface area or electrode surface areas corresponding to the second electrode surface area to the current generator 1716. The measurement component 1718 may measure a second impedance based on the second current, as described above with respect to 1408 of FIG. 14. The current generator 1716 may apply a third current between the active electrode 1730 and the return electrode 1734 in a second direction using a third electrode surface area, for example, as described above with respect to 1410 of FIG. 14. The controller 1714 may control the switching component 1731 to couple the electrode surface area or electrode surface areas corresponding to the third electrode surface area to the current generator 1716. The measurement component 1718 may measure a third impedance based on the third current, as described above with respect to 1412 of FIG. 14. The controller 1714 may determine the pick up ratio or the current in the implantable device based on the first impedance, the second impedance, and the third impedance, for example, as described above with respect to 1414 of FIG. 14.

In some aspects, the external generator 1710 may determine the pick up ratio of the implantable device or the amount of current passing through the implantable device based on measuring impedance between the active electrode 1730 and the return electrode 1734 for current having different amplitudes applied with different electrode surface areas. The current generator 1716 may apply a first current having a first amplitude between the active electrode 1730 and the return electrode 1734 using a first electrode surface area, for example, as described above with respect to 1502 of FIG. 15. The controller 1714 may control the switching component 1731 to couple the electrode surface area or electrode surface areas corresponding to the first electrode surface area to the current generator 1716. The measurement component 1718 may measure a first impedance based on the first current, as described above with respect to 1504 of FIG. 15. The current generator 1716 may apply a second current having the first amplitude between the active electrode 1730 and the return electrode 1734 using a second electrode surface area, for example, as described above with respect to 1506 of FIG. 15. The controller 1714 may control the switching component 1731 to couple the electrode surface area or electrode surface areas corresponding to the second electrode surface area to the current generator 1716. The measurement component 1718 may measure a second impedance based on the second current, as described above with respect to 1508 of FIG. 15. The current generator 1716 may apply a third current having a second amplitude between the active electrode 1730 and the return electrode 1734 using a third electrode surface area, for example, as described above with respect to 1510 of FIG. 15. The controller 1714 may control the switching component 1731 to couple the electrode surface area or electrode surface areas corresponding to the third electrode surface area to the current generator 1716. The measurement component 1718 may measure a third impedance based on the third current, as described above with respect to 1512 of FIG. 15. The controller 1714 may determine the pick up ratio or the current in the implantable device based on the first impedance, the second impedance, and the third impedance, for example, as described above with respect to 1514 of FIG. 15.

In some aspects, the external generator 1710 may determine the pick up ratio of the implantable device or the amount of current passing through the implantable device based on measuring impedance between the active electrode 1730 and the return electrode 1734 for current having different durations applied with different electrode surface areas. The current generator 1716 may apply a first current having a first duration between the active electrode 1730 and the return electrode 1734 using a first electrode surface area, for example, as described above with respect to 1602 of FIG. 16. The controller 1714 may control the switching component 1731 to couple the electrode surface area or electrode surface areas corresponding to the first electrode surface area to the current generator 1716. The measurement component 1718 may measure a first impedance based on the first current, as described above with respect to 1604 of FIG. 16. The current generator 1716 may apply a second current having the first duration between the active electrode 1730 and the return electrode 1734 using a second electrode surface area, for example, as described above with respect to 1606 of FIG. 16. The controller 1714 may control the switching component 1731 to couple the electrode surface area or electrode surface areas corresponding to the second electrode surface area to the current generator 1716. The measurement component 1718 may measure a second impedance based on the second current, as described above with respect to 1608 of FIG. 16. The current generator 1716 may apply a third current having a second duration between the active electrode 1730 and the return electrode 1734 using a third electrode surface area, for example, as described above with respect to 1610 of FIG. 16. The controller 1714 may control the switching component 1731 to couple the electrode surface area or electrode surface areas corresponding to the third electrode surface area to the current generator 1716. The measurement component 1718 may measure a third impedance based on the third current, as described above with respect to 1612 of FIG. 16. The controller 1714 may determine the pick up ratio or the current in the implantable device based on the first impedance, the second impedance, and the third impedance, for example, as described above with respect to 1614 of FIG. 16.

In some aspects, the controller may control the current indication component 1712 to output a current indication based on the determined pick up ratio or implant current, for example as described above with respect to 1412 of FIG. 14, 1512 of FIG. 15, or 1612 of FIG. 16.

In some aspects, the external generator 1710 may be coupled to a remote device 1790. For example, the controller 1714 may wirelessly communicate with the remote device 1790. The remote device 1790 may serve as a user interface for the system. For example, the remote device 1790 may be used to control the external generator 1710, such as turning the application of current on or off, increasing or decreasing the amplitude of applied current, selecting a current program, and/or presenting the user with the implant current indication. In some aspects, the remote device 1790 may include communication components capable of communicating to a cloud-based system, for example, for storage or tracking of patient treatment data. In some aspects, the remote device 1790 may receive data from the external generator 1710 and may perform processing functions for the external generator 1710. For example, the external generator 1710 may transmit the first impedance value, the second impedance value, and the third impedance value to the remote device 1790, the remote device 1790 may determine the implant current or pick up ratio based on the first impedance value, the second impedance value, and the third impedance value, and the remote device 1790 may transmit the implant current or the pick up ratio to the external generator 1710.

By way of example, one or more component of the block diagram discussed above (e.g., the controller 1714), or any portion of a component, or any combination of components may be implemented as a "processing system" that includes one or more processors. Examples of processors include microprocessors, microcontrollers, graphics processing units (GPUs), central processing units (CPUs), application processors, digital signal processors (DSPs), reduced instruction set computing (RISC) processors, systems on a chip (SoC), baseband processors, field programmable gate arrays (FPGAs), programmable logic devices (PLDs), state machines, gated logic, discrete hardware circuits, and other suitable hardware configured to perform the various functionality described throughout this disclosure. One or more processors in the processing system may be coupled to a computer readable medium storing software and may execute the software. Software shall be construed broadly to mean instructions, instruction sets, code, code segments, program code, programs, subprograms, software components, applications, software applications, software packages, routines, subroutines, objects, executables, threads of execution, procedures, functions, etc., whether referred to as software, firmware, middleware, microcode, hardware description language, or otherwise.

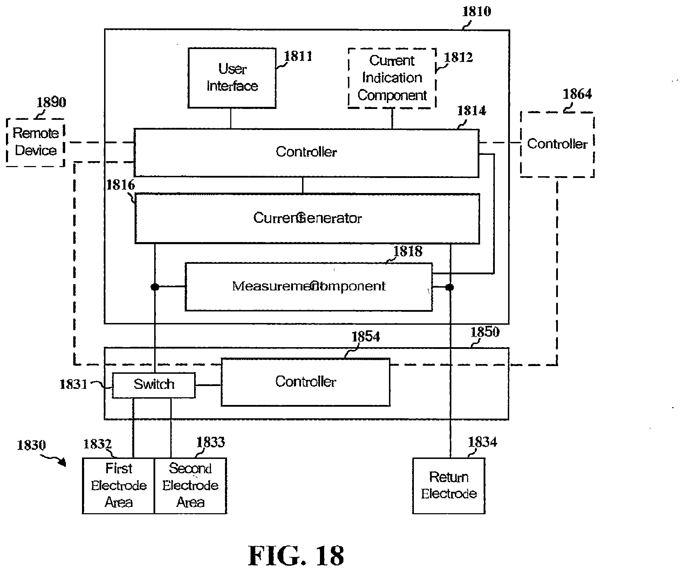

FIG. 18 is a block diagram illustrating an external generator 1810 and an electrode adapter 1850. The external generator 1810 may be the external generator 110 of FIG. 1 or the external generator 510 of FIG. 5. The external generator 1810 includes a current generator 1816, an active electrode 1830, and a return electrode 1834. The external generator 1810 includes a user interface 1811, a current indication component 1812, a controller 1814, a current generator 1816, and a measurement component 1818. The external generator 1810 may apply transcutaneous current to the skin of a subject, and an implantable device beneath the skin of the subject may present an electrical path which allows a portion of the applied transcutaneous current to pass through the implantable device.

The current generator 1816 may be coupled to an active electrode and a return electrode. The current generator 1816 may include a constant current source or a constant voltage source. The current generator 1816 generates the current to be applied to the skin of the subject as transcutaneous current and applies the current between the active electrode and the return electrode. For example, the current generator 1816 may generate bursts of alternating transcutaneous stimulation current and apply the bursts of alternating transcutaneous stimulation current between the active electrode and the return electrode.

The measurement component 1818 is coupled to the active electrode and the return electrode. The measurement component 1818 may measure the impedance between the active electrode and the return electrode. In some aspects, the current generator 1816 may utilize a constant current source to generate the current applied between the active electrode and the return electrode, and the measurement component 1818 may measure the voltage between the active electrode and the return electrode while the current is being applied to measure the impedance between the active electrode and the return electrode. In some aspects, the current generator 1816 may utilize a constant voltage source to generate the current applied between the active electrode and the return electrode, and the measurement component 1818 may measure the current passing between the active electrode and the return electrode while the current is being applied to measure the impedance between the active electrode and the return electrode.

The controller 1814 is coupled to the current generator 1816. The controller 1814 may control when the current generator 1816 applies current between the active electrode and the return electrode. The controller 1814 may control one or more attributes of the current applied by the current generator 1816. For example, the current generator 1816 may apply bursts of alternating current between the active electrode and the return electrode, and the controller 1814 may set any of the amplitude, the carrier frequency, the burst duration, and the burst frequency of the current, or any combination thereof.

The controller 1814 is coupled to the measurement component 1818. The controller 1814 may control when the measurement component 1818 takes impedance measurements between the active electrode and the return electrode.

In some aspects, the controller 1814 may be coupled to a current indication component 1812. The current indication component 1812 may be an element on the user interface 1811, may be an LED or other light, or may be a speaker. The controller 1814 may control the current indication component 1812 to output a current indication, as discussed further below.

The electrode adapter 1850 is capable of enabling the external generator 1810 to apply transcutaneous current between the active electrode 1830 and the return electrode 1834 utilizing different electrode surface areas. The electrode adapter 1850 includes a controller 1854, a switching component 1831, an active electrode 1830, and a return electrode 1834. During normal operation (e.g., applying therapeutic transcutaneous stimulation to the skin of a subject), the external generator 1810 may be attached to an active electrode and a return electrode with singular surface areas. When it is desired for the external generator 1810 to be able to utilize multiple electrode surface areas (e.g., to determine the pick up ratio of the implantable device or the current passing through the implantable device), the external generator 1810 may be attached to the electrode adapter 1850. The active electrode 1830 coupled to the electrode adapter 1850 includes a first electrode area 1832 and a second electrode area 1833. The first electrode area 1832 and the second electrode area 1833 may be electrically separate. The switching component 1831 is coupled between the first electrode area 1832 and the current generator 1816 and is coupled between the second electrode area 1833 and the current generator 1816. The switching component 1831 may control whether the first electrode area 1832 and the second electrode area 1833 are electrically coupled to the current generator 1816. For example, the switching component 1831 may include one or more mechanical switch or one or more transistor. The controller 1854 of the electrode adapter 1850 is coupled to the switching component 1831 and may control which electrode areas of the active electrode 1830 the switching component 1831 electrically couples to the current generator 1816.

The external generator 1810 may determine the pick up ratio of the implantable device or the amount of current passing through the implantable device without feedback from the implantable device based on measuring impedance between the active electrode 1830 and the return electrode 1834 utilizing different electrode surface areas. In some aspects, the external generator 1810 may determine the pick up ratio of the implantable device or the amount of current passing through the implantable device based on measuring impedances for current applied in different directions with different electrode surface areas. In some aspects, the external generator 1810 may determine the pick up ratio of the implantable device or the amount of current passing through the implantable device based on measuring impedances for current applied having different amplitudes with different electrode surface areas. In some aspects, the external generator 1810 may determine the pick up ratio of the implantable device or the amount of current passing through the implantable device based on measuring impedances for current applied having different durations with different electrode surface areas.

The current generator 1816 of the external generator 1810 may apply a first current between the active electrode 1830 and the return electrode 1834 using a first electrode surface area, for example, as described above with respect to 1402 of FIG. 14, 1502 of FIG. 15, or 1602 of FIG. 16. In some aspects, the first current may be in a first direction, for example, as described above with respect to 1402 of FIG. 14. In some aspects, the first current may have a first amplitude, as described above with respect to 1502 of FIG. 15. In some aspects, the first current may have a first duration, as described above with respect to 1602 of FIG. 16. The controller 1854 of the electrode adapter 1850 may control the switching component 1831 to couple the electrode surface area or electrode surface areas corresponding to the first electrode surface area to the current generator 1816. In some aspects, the controller 1814 of the external generator 1810 may indicate to the controller 1854 of the electrode adapter 1850 to utilize the first electrode surface area, and the controller 1854 may utilize the first electrode surface area based on the indication. In some aspects, a separate controller 1864 may indicate to the controller 1814 of the external generator 1810 and to the controller 1854 of the electrode adapter 1850 to utilize the first electrode surface area, and the controller 1854 may utilize the first electrode surface area based on the indication. In some aspects, the controller 1854 of the electrode adapter 1850 may control the switching component 1831 based on a pre-defined sequence. The measurement component 1818 may measure a first impedance based on the first current, for example, as described above with respect to 1404 of FIG. 14, 1504 of FIG. 15, or 1604 of FIG. 16.

The current generator 1816 of the external generator 1810 may apply a second current between the active electrode 1830 and the return electrode 1834 using a second electrode surface area, for example, as described above with respect to 1406 of FIG. 14, 1506 of FIG. 15, or 1606 of FIG. 16. In some aspects, the second current may be in the first direction, for example, as described above with respect to 1406 of FIG. 14. In some aspects, the second current may have the first amplitude, as described above with respect to 1506 of FIG. 15. In some aspects, the second current may have the first duration, as described above with respect to 1606 of FIG. 16. The controller 1854 of the electrode adapter 1850 may control the switching component 1831 to couple the electrode surface area or electrode surface areas corresponding to the second electrode surface area to the current generator 1816. In some aspects, the controller 1814 of the external generator 1810 may indicate to the controller 1854 of the electrode adapter 1850 to utilize the second electrode surface area, and the controller 1854 may utilize the second electrode surface area based on the indication. In some aspects, a separate controller 1864 may indicate to the controller 1814 of the external generator 1810 and to the controller 1854 of the electrode adapter 1850 to utilize the second electrode surface area, and the controller 1854 may utilize the second electrode surface area based on the indication. The measurement component 1818 may measure a second impedance based on the second current, for example, as described above with respect to 1404 of FIG. 14, 1504 of FIG. 15, or 1604 of FIG. 16.

The current generator 1816 of the external generator 1810 may apply a third current between the active electrode 1830 and the return electrode 1834 using a third electrode surface area, for example, as described above with respect to 1410 of FIG. 14, 1510 of FIG. 15, or 1610 of FIG. 16. In some aspects, the third current may be in a second direction, for example, as described above with respect to 1410 of FIG. 14. In some aspects, the third current may have a second amplitude, as described above with respect to 1510 of FIG. 15. In some aspects, the third current may have a second duration, as described above with respect to 1610 of FIG. 16. The controller 1854 of the electrode adapter 1850 may control the switching component 1831 to couple the electrode surface area or electrode surface areas corresponding to the third electrode surface area to the current generator 1816. In some aspects, the controller 1814 of the external generator 1810 may indicate to the controller 1854 of the electrode adapter 1850 to utilize the third electrode surface area, and the controller 1854 may utilize the third electrode surface area based on the indication. In some aspects, a separate controller 1864 may indicate to the controller 1814 of the external generator 1810 and to the controller 1854 of the electrode adapter 1850 to utilize the third electrode surface area, and the controller 1854 may utilize the third electrode surface area based on the indication. The measurement component 1818 may measure a third impedance based on the third current, for example, as described above with respect to 1410 of FIG. 14, 1510 of FIG. 15, or 1610 of FIG. 16.

In some aspects, the external generator 1810 and/or the electrode adapter 1850 may be coupled to a remote device 1890. For example, the external generator 1810 or the controller 1814 may wirelessly communicate with the remote device 1890. The remote device 1890 may serve as a user interface for the system. For example, the remote device 1890 may be used to control the external generator 1810, such as turning the application of current on or off, increasing or decreasing the amplitude of applied current, selecting a current program, and/or presenting the user with the functionality indication. In some aspects, the remote device 1890 may include communication components capable of communicating to a cloud-based system, for example, for storage or tracking of patient treatment data. In some aspects, the remote device 1890 may receive data from the external generator 1810 or the electrode adapter 1850 and may perform processing functions for the external generator 1810 or the electrode adapter 1850. For example, the external generator 1810 or the electrode adapter 1850 may transmit the first impedance value, the second impedance value, and the third impedance value to the remote device 1890, the remote device 1890 may determine the implant current or pick up ratio based on the first impedance value, the second impedance value, and the third impedance value, and the remote device 1890 may transmit the implant functionality to the external generator 1810 or the electrode adapter 1850.

By way of example, one or more component of the block diagram discussed above (e.g., the controller 1814 or the controller 1854), or any portion of a component, or any combination of components may be implemented as a "processing system" that includes one or more processors. Examples of processors include microprocessors, microcontrollers, graphics processing units (GPUs), central processing units (CPUs), application processors, digital signal processors (DSPs), reduced instruction set computing (RISC) processors, systems on a chip (SoC), baseband processors, field programmable gate arrays (FPGAs), programmable logic devices (PLDs), state machines, gated logic, discrete hardware circuits, and other suitable hardware configured to perform the various functionality described throughout this disclosure. One or more processors in the processing system may be coupled to a computer readable medium storing software and may execute the software. Software shall be construed broadly to mean instructions, instruction sets, code, code segments, program code, programs, subprograms, software components, applications, software applications, software packages, routines, subroutines, objects, executables, threads of execution, procedures, functions, etc., whether referred to as software, firmware, middleware, microcode, hardware description language, or otherwise.

It is understood that the specific order or hierarchy of blocks in the processes / flowcharts disclosed is an illustration of example approaches. Based upon design preferences, it is understood that the specific order or hierarchy of blocks in the processes / flowcharts may be rearranged. Further, some blocks may be combined or omitted. The accompanying method claims present elements of the various blocks in a sample order, and are not meant to be limited to the specific order or hierarchy presented.

The previous description is provided to enable any person skilled in the art to practice the various aspects described herein. Various modifications to these aspects will be readily apparent to those skilled in the art, and the generic principles defined herein may be applied to other aspects. Thus, the claims are not intended to be limited to the aspects shown herein, but is to be accorded the full scope consistent with the language claims, wherein reference to an element in the singular is not intended to mean "one and only one" unless specifically so stated, but rather "one or more." The word "exemplary" is used herein to mean "serving as an example, instance, or illustration." Any aspect described herein as "exemplary" is not necessarily to be construed as preferred or advantageous over other aspects. Unless specifically stated otherwise, the term "some" refers to one or more. Combinations such as "at least one of A, B, or C," "one or more of A, B, or C," "at least one of A, B, and C," "one or more of A, B, and C," and "A, B, C, or any combination thereof" include any combination of A, B, and/or C, and may include multiples of A, multiples of B, or multiples of C. Specifically, combinations such as "at least one of A, B, or C," "one or more of A, B, or C," "at least one of A, B, and C," "one or more of A, B, and C," and "A, B, C, or any combination thereof" may be A only, B only, C only, A and B, A and C, B and C, or A and B and C, where any such combinations may contain one or more member or members of A, B, or C.. Moreover, nothing disclosed herein is intended to be dedicated to the public regardless of whether such disclosure is explicitly recited in the claims. The words "module," "mechanism," "element," "device," and the like may not be a substitute for the word "means." As such, no claim element is to be construed as a means plus function unless the element is expressly recited using the phrase "means for."

## Claims

1. A method of evaluating a coupling between an implantable device (130) and an external generator (110, 1710), the implantable device (130) being implanted beneath the skin of a subject, the external generator (110, 1710) comprising a first skin electrode (114, 1730) and a second skin electrode (116, 1734) in contact with the skin of the subject, the method comprising:
applying a first transcutaneous current to the skin of the subject between the first skin electrode (114, 1730) and the second skin electrode (116. 1734) utilizing a first electrode surface area (1732);
determining a first impedance value based on the first current;
applying a second transcutaneous current to the skin of the subject between the first skin electrode (114, 1730) and the second skin electrode (116, 1734) utilizing a second electrode surface area (1733);
determining a second impedance value based on the second current;
applying a third transcutaneous current to the skin of the subject between the first skin electrode (114, 1730) and the second skin electrode (116, 1734) utilizing a third electrode surface area, the third transcutaneous current being different than the first transcutaneous current;
determining a third impedance value based on the third current; and
determining an implant current or a pick up ratio of the implantable device (130) based on the first impedance value, the second impedance value, and the third impedance value.

2. The method of claim 1, wherein the first transcutaneous current is applied in a first direction, the second transcutaneous current is applied in the first direction, and the third transcutaneous current is applied in a second direction different than the first direction.

3. The method of claim 2, wherein the implantable device (130) comprises a rectifier (200), the first transcutaneous current does not cause the rectifier (200) to conduct based on the first direction, the second transcutaneous current does not cause the rectifier (200) to conduct based on the first direction, and the third transcutaneous current does cause the rectifier (200) to conduct based on the second direction.

4. The method of claim 1, wherein the first transcutaneous current and the second transcutaneous current have a first amplitude, and the third transcutaneous current has a second amplitude, the second amplitude being different than the first amplitude.

5. The method of claim 1, wherein the first transcutaneous current is applied for a first duration, the second transcutaneous current is applied for the first duration, and the third transcutaneous current is applied for a second duration, the second duration being different than the first duration.

6. The method of claim 5, wherein:
the implantable device (130) comprises a capacitor (222) that may be charged by current passing through the implantable device (130),
applying the first transcutaneous current over the first duration causes the capacitor (222) to have a first effective impedance,
applying the second transcutaneous current over the first duration causes the capacitor (222) to have a second effective impedance,
applying the third transcutaneous current over the second duration causes the capacitor (222) to have a third effective impedance, and
the third effective impedance is lower than the first effective impedance and the second effective impedance.

7. The method of claim 1, wherein determining the implant current or the pick up ratio of the implantable device (130) comprises determining a first electrode-skin impedance value corresponding to the first electrode surface area (1732), a second electrode-skin impedance value corresponding to the second electrode surface area (1733), a tissue path impedance value (Rt), and an implant path impedance value (Rti, Ri).

8. The method of claim 7, wherein determining the first electrode-skin impedance value or the second electrode-skin impedance value comprises determining a ratio between the first electrode surface area (1732) and the second electrode surface area (1733) based on a capacitive component of the skin of the subject.

9. An external generator (1010, 1710), comprising:
a current generator (1016, 1716);
a first skin electrode (1032, 1730) coupled to the current generator (1016, 1716);
a second skin electrode (1034, 1734) coupled to the current generator (1016, 1716); and
at least one processor (1014, 1714) coupled to the current generator (1016, 1716), wherein:
the current generator (1016, 1716) is configured to apply a first transcutaneous current to a skin of a subject between the first skin electrode (1032, 1730) and the second skin electrode (1034, 1734) utilizing a first electrode surface area (1732),
the processor (1014, 1714) is configured to determine a first impedance value based on the first current,
the current generator (1016, 1716) is configured to apply a second transcutaneous current to the skin of the subject between the first skin electrode (1032, 1730) and the second skin electrode (1034, 1734) utilizing a second electrode surface area (1733),
the processor (1014, 1714) is configured to determine a second impedance value based on the second current,
the current generator (1016, 1716) is configured to apply a third transcutaneous current to the skin of the subject between the first skin electrode (1032, 1730) and the second skin electrode (1034, 1734) utilizing a third electrode surface area, the third transcutaneous current being different than the first transcutaneous current,
the processor (1014, 1714) is configured to determine a third impedance value based on the third current, and
the processor (1014, 1714) is configured to determine an implant current or a pick up ratio of an implantable device (130) implanted beneath the skin of the subject based on the first impedance value, the second impedance value, and the third impedance value

10. The external generator (1010, 1710) of claim 9, wherein the current generator (1016, 1716) is configured to apply the first transcutaneous current in a first direction, apply the second transcutaneous current in the first direction, and apply the third transcutaneous current in a second direction different than the first direction.

11. The external generator (1010, 1710) of claim 9, wherein the first transcutaneous current and the second transcutaneous current have a first amplitude, and the third transcutaneous current has a second amplitude, the second amplitude being different than the first amplitude.

12. The external generator (1010, 1710) of claim 9, wherein the current generator (1016, 1716) is configured to apply the first transcutaneous current for a first duration, apply the second transcutaneous current for the first duration, and apply the third transcutaneous current for a second duration, the second duration being different than the first duration.

13. The external generator (1010, 1710) of claim 12, wherein:
the implantable device (130) comprises a capacitor (222) that may be charged by current passing through the implantable device (130),
applying the first transcutaneous current over the first duration causes the capacitor (222) to have a first effective impedance,
applying the second transcutaneous current over the first duration causes the capacitor (222) to have a second effective impedance,
applying the third transcutaneous current over the second duration causes the capacitor (222) to have a third effective impedance, and
the third effective impedance is lower than the first effective impedance and the second effective impedance.

14. The external generator (1010, 1710) of claim 9, wherein determining the implant current or the pick up ratio of the implantable device (130) comprises determining a first electrode-skin impedance value corresponding to the first electrode surface area (1732), a second electrode-skin impedance value corresponding to the second electrode surface area (1733), a tissue path impedance value (Rt), and an implant path impedance value (Rti, Ri).

15. The external generator (1010, 1710) of claim 14, wherein determining the first electrode-skin impedance value or the second electrode-skin impedance value comprises determining a ratio between the first electrode surface area (1732) and the second electrode surface area (1733) based on a capacitive component of the skin of the subject.

## Patentansprüche

1. Ein Verfahren zum Beurteilen einer Kopplung zwischen einer implantierbaren Vorrichtung (130) und einem externen Generator (110, 1710), wobei die implantierbare Vorrichtung (130) unter der Haut eines Individuums implantiert ist, wobei der externe Generator (110, 1710) eine erste Hautelektrode (114, 1730) und eine zweite Hautelektrode (116, 1734) in Kontakt mit der Haut des Individuums beinhaltet, wobei das Verfahren Folgendes beinhaltet:
Anlegen eines ersten transkutanen Stroms an die Haut des Individuums zwischen der ersten Hautelektrode (114, 1730) und der zweiten Hautelektrode (116, 1734) unter Nutzung eines ersten Elektrodenoberflächenbereichs (1732);
Bestimmen eines ersten Impedanzwerts auf der Basis des ersten Stroms;
Anlegen eines zweiten transkutanen Stroms an die Haut des Individuums zwischen der ersten Hautelektrode (114, 1730) und der zweiten Hautelektrode (116, 1734) unter Nutzung eines zweiten Elektrodenoberflächenbereichs (1733);
Bestimmen eines zweiten Impedanzwerts auf der Basis des zweiten Stroms;
Anlegen eines dritten transkutanen Stroms an die Haut des Individuums zwischen der ersten Hautelektrode (114, 1730) und der zweiten Hautelektrode (116, 1734) unter Nutzung eines dritten Elektrodenoberflächenbereichs, wobei sich der dritte transkutane Strom von dem ersten transkutanen Strom unterscheidet;
Bestimmen eines dritten Impedanzwerts auf der Basis des dritten Stroms; und
Bestimmen eines Implantatstroms oder eines Aufnahmeverhältnisses der implantierbaren Vorrichtung (130) auf der Basis des ersten Impedanzwerts, des zweiten Impedanzwerts und des dritten Impedanzwerts.

2. Verfahren gemäß Anspruch 1, wobei der erste transkutane Strom in einer ersten Richtung angelegt wird, der zweite transkutane Strom in der ersten Richtung angelegt wird und der dritte transkutane Strom in einer zweiten Richtung angelegt wird, die sich von der ersten Richtung unterscheidet.

3. Verfahren gemäß Anspruch 2, wobei die implantierbare Vorrichtung (130) einen Gleichrichter (200) beinhaltet, der erste transkutane Strom auf der Basis der ersten Richtung nicht bewirkt, dass der Gleichrichter (200) leitet, der zweite transkutane Strom auf der Basis der ersten Richtung nicht bewirkt, dass der Gleichrichter (200) leitet, und der dritte transkutane Strom auf der Basis der zweiten Richtung bewirkt, dass der Gleichrichter (200) leitet.

4. Verfahren gemäß Anspruch 1, wobei der erste transkutane Strom und der zweite transkutane Strom eine erste Amplitude aufweisen und der dritte transkutane Strom eine zweite Amplitude aufweist, wobei sich die zweite Amplitude von der ersten Amplitude unterscheidet.

5. Verfahren gemäß Anspruch 1, wobei der erste transkutane Strom für eine erste Dauer angelegt wird, der zweite transkutane Strom für die erste Dauer angelegt wird und der dritte transkutane Strom für eine zweite Dauer angelegt wird, wobei sich die zweite Dauer von der ersten Dauer unterscheidet.

6. Verfahren gemäß Anspruch 5, wobei:
die implantierbare Vorrichtung (130) einen Kondensator (222) beinhaltet, der durch Strom geladen werden kann, welcher durch die implantierbare Vorrichtung (130) fließt,
das Anlegen des ersten transkutanen Stroms über die erste Dauer bewirkt, dass der Kondensator (222) eine erste effektive Impedanz aufweist,
das Anlegen des zweiten transkutanen Stroms über die erste Dauer bewirkt, dass der Kondensator (222) eine zweite effektive Impedanz aufweist,
das Anlegen des dritten transkutanen Stroms über die zweite Dauer bewirkt, dass der Kondensator (222) eine dritte effektive Impedanz aufweist, und
die dritte effektive Impedanz niedriger ist als die erste effektive Impedanz und die zweite effektive Impedanz.

7. Verfahren gemäß Anspruch 1, wobei das Bestimmen des Implantatstroms oder des Aufnahmeverhältnisses der implantierbaren Vorrichtung (130) das Bestimmen eines ersten Elektroden-Haut-Impedanzwerts, der dem ersten Elektrodenoberflächenbereich (1732) entspricht, eines zweiten Elektroden-Haut-Impedanzwerts, der dem zweiten Elektrodenoberflächenbereich (1733) entspricht, eines Gewebepfadimpedanzwerts (Rt) und eines Implantatpfadimpedanzwerts (Rti, Ri) beinhaltet.

8. Verfahren gemäß Anspruch 7, wobei das Bestimmen des ersten Elektroden-Haut-Impedanzwerts oder des zweiten Elektroden-Haut-Impedanzwerts das Bestimmen eines Verhältnisses zwischen dem ersten Elektrodenoberflächenbereich (1732) und dem zweiten Elektrodenoberflächenbereich (1733) auf der Basis einer kapazitiven Komponente der Haut des Individuums beinhaltet.

9. Ein externer Generator (1010, 1710), der Folgendes beinhaltet:
einen Stromgenerator (1016, 1716);
eine erste Hautelektrode (1032, 1730), die mit dem Stromgenerator (1016, 1716) gekoppelt ist;
eine zweite Hautelektrode (1034, 1734), die mit dem Stromgenerator (1016, 1716) gekoppelt ist; und
mindestens einen Prozessor (1014, 1714), der mit dem Stromgenerator (1016, 1716) gekoppelt ist, wobei:
der Stromgenerator (1016, 1716) konfiguriert ist, um an eine Haut eines Individuums zwischen der ersten Hautelektrode (1032, 1730) und der zweiten Hautelektrode (1034, 1734) unter Nutzung eines ersten Elektrodenoberflächenbereichs (1732) einen ersten transkutanen Strom anzulegen,
der Prozessor (1014, 1714) konfiguriert ist, um auf der Basis des ersten Stroms einen ersten Impedanzwert zu bestimmen,
der Stromgenerator (1016, 1716) konfiguriert ist, um an die Haut des Individuums zwischen der ersten Hautelektrode (1032, 1730) und der zweiten Hautelektrode (1034, 1734) unter Nutzung eines zweiten Elektrodenoberflächenbereichs (1733) einen zweiten transkutanen Strom anzulegen,
der Prozessor (1014, 1714) konfiguriert ist, um auf der Basis des zweiten Stroms einen zweiten Impedanzwert zu bestimmen,
der Stromgenerator (1016, 1716) konfiguriert ist, um an die Haut des Individuums zwischen der ersten Hautelektrode (1032, 1730) und der zweiten Hautelektrode (1034, 1734) unter Nutzung eines dritten Elektrodenoberflächenbereichs einen dritten transkutanen Strom anzulegen, wobei sich der dritte transkutane Strom von dem ersten transkutanen Strom unterscheidet,
der Prozessor (1014, 1714) konfiguriert ist, um auf der Basis des dritten Stroms einen dritten Impedanzwert zu bestimmen, und
der Prozessor (1014, 1714) konfiguriert ist, um auf der Basis des ersten Impedanzwerts, des zweiten Impedanzwerts und des dritten Impedanzwerts einen Implantatstrom oder ein Aufnahmeverhältnis einer implantierbaren Vorrichtung (130) zu bestimmen, die unter der Haut des Individuums implantiert ist.

10. Externer Generator (1010, 1710) gemäß Anspruch 9, wobei der Stromgenerator (1016, 1716) konfiguriert ist, um den ersten transkutanen Strom in einer ersten Richtung anzulegen, den zweiten transkutanen Strom in der ersten Richtung anzulegen und den dritten transkutanen Strom in einer zweiten Richtung anzulegen, die sich von der ersten Richtung unterscheidet.

11. Externer Generator (1010, 1710) gemäß Anspruch 9, wobei der erste transkutane Strom und der zweite transkutane Strom eine erste Amplitude aufweisen und der dritte transkutane Strom eine zweite Amplitude aufweist, wobei sich die zweite Amplitude von der ersten Amplitude unterscheidet.

12. Externer Generator (1010, 1710) gemäß Anspruch 9, wobei der Stromgenerator (1016, 1716) konfiguriert ist, um den ersten transkutanen Strom für eine erste Dauer anzulegen, den zweiten transkutanen Strom für die erste Dauer anzulegen und den dritten transkutanen Strom für eine zweite Dauer anzulegen, wobei sich die zweite Dauer von der ersten Dauer unterscheidet.

13. Externer Generator (1010, 1710) gemäß Anspruch 12, wobei:
die implantierbare Vorrichtung (130) einen Kondensator (222) beinhaltet, der durch Strom geladen werden kann, welcher durch die implantierbare Vorrichtung (130) fließt,
das Anlegen des ersten transkutanen Stroms über die erste Dauer bewirkt, dass der Kondensator (222) eine erste effektive Impedanz aufweist,
das Anlegen des zweiten transkutanen Stroms über die erste Dauer bewirkt, dass der Kondensator (222) eine zweite effektive Impedanz aufweist,
das Anlegen des dritten transkutanen Stroms über die zweite Dauer bewirkt, dass der Kondensator (222) eine dritte effektive Impedanz aufweist, und
die dritte effektive Impedanz niedriger ist als die erste effektive Impedanz und die zweite effektive Impedanz.

14. Externer Generator (1010, 1710) gemäß Anspruch 9, wobei das Bestimmen des Implantatstroms oder des Aufnahmeverhältnisses der implantierbaren Vorrichtung (130) das Bestimmen eines ersten Elektroden-Haut-Impedanzwerts, der dem ersten Elektrodenoberflächenbereich (1732) entspricht, eines zweiten Elektroden-Haut-Impedanzwerts, der dem zweiten Elektrodenoberflächenbereich (1733) entspricht, eines Gewebepfadimpedanzwerts (Rt) und eines Implantatpfadimpedanzwerts (Rti, Ri) beinhaltet.

15. Externer Generator (1010, 1710) gemäß Anspruch 14, wobei das Bestimmen des ersten Elektroden-Haut-Impedanzwerts oder des zweiten Elektroden-Haut-Impedanzwerts das Bestimmen eines Verhältnisses zwischen dem ersten Elektrodenoberflächenbereich (1732) und dem zweiten Elektrodenoberflächenbereich (1733) auf der Basis einer kapazitiven Komponente der Haut des Individuums beinhaltet.

## Revendications

1. Un procédé d'évaluation d'un couplage entre un dispositif implantable (130) et un générateur externe (110, 1710), le dispositif implantable (130) étant implanté sous la peau d'un sujet, le générateur externe (110, 1710) comprenant une première électrode cutanée (114, 1730) et une deuxième électrode cutanée (116, 1734) en contact avec la peau du sujet, le procédé comprenant :
l'application d'un premier courant transcutané à la peau du sujet entre la première électrode cutanée (114, 1730) et la deuxième électrode cutanée (116, 1734) en employant une première superficie d'électrode (1732) ;
la détermination d'une première valeur d'impédance sur la base du premier courant ;
l'application d'un deuxième courant transcutané à la peau du sujet entre la première électrode cutanée (114, 1730) et la deuxième électrode cutanée (116, 1734) en employant une deuxième superficie d'électrode (1733) ;
la détermination d'une deuxième valeur d'impédance sur la base du deuxième courant ;
l'application d'un troisième courant transcutané à la peau du sujet entre la première électrode cutanée (114, 1730) et la deuxième électrode cutanée (116, 1734) en employant une troisième superficie d'électrode, le troisième courant transcutané étant différent du premier courant transcutané ;
la détermination d'une troisième valeur d'impédance sur la base du troisième courant ; et
la détermination d'un courant d'implant ou d'un rapport de captage du dispositif implantable (130) sur la base de la première valeur d'impédance, de la deuxième valeur d'impédance et de la troisième valeur d'impédance.

2. Le procédé de la revendication 1, dans lequel le premier courant transcutané est appliqué dans un premier sens, le deuxième courant transcutané est appliqué dans le premier sens, et le troisième courant transcutané est appliqué dans un deuxième sens différent du premier sens.

3. Le procédé de la revendication 2, dans lequel le dispositif implantable (130) comprend un redresseur (200), le premier courant transcutané ne fait pas conduire le redresseur (200) sur la base du premier sens, le deuxième courant transcutané ne fait pas conduire le redresseur (200) sur la base du premier sens, et le troisième courant transcutané fait conduire le redresseur (200) sur la base du deuxième sens.

4. Le procédé de la revendication 1, dans lequel le premier courant transcutané et le deuxième courant transcutané ont une première amplitude, et le troisième courant transcutané a une deuxième amplitude, la deuxième amplitude étant différente de la première amplitude.

5. Le procédé de la revendication 1, dans lequel le premier courant transcutané est appliqué pendant une première durée, le deuxième courant transcutané est appliqué pendant la première durée, et le troisième courant transcutané est appliqué pendant une deuxième durée, la deuxième durée étant différente de la première durée.

6. Le procédé de la revendication 5, dans lequel :
le dispositif implantable (130) comprend un condensateur (222) qui peut être chargé par un courant traversant le dispositif implantable (130),
l'application du premier courant transcutané durant la première durée confère au condensateur (222) une première impédance effective,
l'application du deuxième courant transcutané durant la première durée confère au condensateur (222) une deuxième impédance effective,
l'application du troisième courant transcutané durant la deuxième durée confère au condensateur (222) une troisième impédance effective, et
la troisième impédance effective est inférieure à la première impédance effective et à la deuxième impédance effective.

7. Le procédé de la revendication 1, dans lequel la détermination du courant d'implant ou du rapport de captage du dispositif implantable (130) comprend la détermination d'une première valeur d'impédance électrode-peau correspondant à la première superficie d'électrode (1732), d'une deuxième valeur d'impédance électrode-peau correspondant à la deuxième superficie d'électrode (1733), d'une valeur d'impédance de trajet tissulaire (Rt) et d'une valeur d'impédance de trajet d'implant (Rti, Ri).

8. Le procédé de la revendication 7, dans lequel la détermination de la première valeur d'impédance électrode-peau ou de la deuxième valeur d'impédance électrode-peau comprend la détermination d'un rapport entre la première superficie d'électrode (1732) et la deuxième superficie d'électrode (1733) sur la base d'une composante capacitive de la peau du sujet.

9. Un générateur externe (1010, 1710), comprenant :
un générateur de courant (1016, 1716) ;
une première électrode cutanée (1032, 1730) couplée au générateur de courant (1016, 1716) ;
une deuxième électrode cutanée (1034, 1734) couplée au générateur de courant (1016, 1716) ; et
au moins un processeur (1014, 1714) couplé au générateur de courant (1016, 1716),
dans lequel :
le générateur de courant (1016, 1716) est configuré pour appliquer un premier courant transcutané à la peau d'un sujet entre la première électrode cutanée (1032, 1730) et la deuxième électrode cutanée (1034, 1734) en employant une première superficie d'électrode (1732),
le processeur (1014, 1714) est configuré pour déterminer une première valeur d'impédance sur la base du premier courant,
le générateur de courant (1016, 1716) est configuré pour appliquer un deuxième courant transcutané à la peau du sujet entre la première électrode cutanée (1032, 1730) et la deuxième électrode cutanée (1034, 1734) en employant une deuxième superficie d'électrode (1733),
le processeur (1014, 1714) est configuré pour déterminer une deuxième valeur d'impédance sur la base du deuxième courant,
le générateur de courant (1016, 1716) est configuré pour appliquer un troisième courant transcutané à la peau du sujet entre la première électrode cutanée (1032, 1730) et la deuxième électrode cutanée (1034, 1734) en employant une troisième superficie d'électrode, le troisième courant transcutané étant différent du premier courant transcutané,
le processeur (1014, 1714) est configuré pour déterminer une troisième valeur d'impédance sur la base du troisième courant, et
le processeur (1014, 1714) est configuré pour déterminer un courant d'implant ou un rapport de captage d'un dispositif implantable (130) implanté sous la peau du sujet sur la base de la première valeur d'impédance, de la deuxième valeur d'impédance et de la troisième valeur d'impédance.

10. Le générateur externe (1010, 1710) de la revendication 9, dans lequel le générateur de courant (1016, 1716) est configuré pour appliquer le premier courant transcutané dans un premier sens, appliquer le deuxième courant transcutané dans le premier sens, et appliquer le troisième courant transcutané dans un deuxième sens différent du premier sens.

11. Le générateur externe (1010, 1710) de la revendication 9, dans lequel le premier courant transcutané et le deuxième courant transcutané ont une première amplitude, et le troisième courant transcutané a une deuxième amplitude, la deuxième amplitude étant différente de la première amplitude.

12. Le générateur externe (1010, 1710) de la revendication 9, dans lequel le générateur de courant (1016, 1716) est configuré pour appliquer le premier courant transcutané pendant une première durée, appliquer le deuxième courant transcutané pendant la première durée, et appliquer le troisième courant transcutané pendant une deuxième durée, la deuxième durée étant différente de la première durée.

13. Le générateur externe (1010, 1710) de la revendication 12, dans lequel :
le dispositif implantable (130) comprend un condensateur (222) qui peut être chargé par un courant traversant le dispositif implantable (130),
l'application du premier courant transcutané durant la première durée confère au condensateur (222) une première impédance effective,
l'application du deuxième courant transcutané durant la première durée confère au condensateur (222) une deuxième impédance effective,
l'application du troisième courant transcutané durant la deuxième durée confère au condensateur (222) une troisième impédance effective, et
la troisième impédance effective est inférieure à la première impédance effective et à la deuxième impédance effective.

14. Le générateur externe (1010, 1710) de la revendication 9, dans lequel la détermination du courant d'implant ou du rapport de captage du dispositif implantable (130) comprend la détermination d'une première valeur d'impédance électrode-peau correspondant à la première superficie d'électrode (1732), d'une deuxième valeur d'impédance électrode-peau correspondant à la deuxième superficie d'électrode (1733), d'une valeur d'impédance de trajet tissulaire (Rt) et d'une valeur d'impédance de trajet d'implant (Rti, Ri).

15. Le générateur externe (1010, 1710) de la revendication 14, dans lequel la détermination de la première valeur d'impédance électrode-peau ou de la deuxième valeur d'impédance électrode-peau comprend la détermination d'un rapport entre la première superficie d'électrode (1732) et la deuxième superficie d'électrode (1733) sur la base d'une composante capacitive de la peau du sujet.
